# EUROPEAN PATENT APPLICATION

(11) **EP 4 737 591 A1**
(43) Date of publication of application: **06.05.2026**
(21) Application number: 24210955.1
(22) Date of filing: 05.11.2024
(51) Int. Cl.: C12Q 1/6886, G01N 33/574

(54) **METHOD AND KIT FOR DIAGNOSIS, PROGNOSIS, AND/OR STRATIFICATION OF DIFFUSE LARGE B-CELL LYMPHOMA (DLBCL)**

(71) Applicant: Johann-Wolfgang-Goethe-Universität Frankfurt am Main, 60323 Frankfurt am Main (DE); Deutsches Krebsforschungszentrum Stiftung des öffentlichen Rechts, 69120 Heidelberg (DE)
(72) Inventor: The designation of the inventor has not yet been filed
(74) Representative: Engelhard, Markus

(57) **Abstract**

The present invention relates to a method and a kit for the diagnosis, prognosis, and/or stratification of diffuse large B-cell lymphoma (DLBCL).

## Description

### FIELD OF THE INVENTION

The present invention relates to a method and a kit for the diagnosis, prognosis, and/or stratification of diffuse large B-cell lymphoma (DLBCL).

### BACKGROUND OF THE INVENTION

Diffuse large B-cell lymphoma (DLBCL) is a genetically and clinically heterogenous disease. While about 60-70% of DLCBL patients can be cured with R-CHOP chemoimmunotherapy, 30-40% have either primary refractory disease or relapse after treatment. Gene expression profiling was the first step towards resolving this clinical heterogeneity. Two dominant gene expression subtypes were identified: the germinal center B cell-like (GCB) and activated B cell-like (ABC) lymphomas, expressing a gene expression program akin to either GC B cells or B cells activated through their B-cell antigen receptor, respectively. The fact that 10-15% of DLBCL remain unclassifiable by gene expression profiling and that several clinical studies revealed discrepant clinical outcome of the individual cell-of-origin subtypes point towards further molecular heterogeneity. Therefore, subsequent studies have elucidated the genomic landscape of DLBCL and established a genetic classification with seven major genetic subtypes, each being characterized by a specific pattern of genomic alterations. Distinct genetic profiles were found to be associated with the ABC or GCB gene expression profiles. For instance, tumors with mutations in the B-cell receptor (BCR) subunit CD79B and the immune signaling adapter protein MYD88 (the MCD/C5 genetic subtype) were identified as the most prevalent form of ABC-DLBCL. In contrast, mutations in the EZH2 gene and translocations of BCL2 were mainly found in GCB tumors. Importantly, some genetic alterations are linked to specific survival pathways that can be therapeutically exploited. The ABC-MCD-DLBCL subtype relies on chronic active BCR/NF-κB signaling and is sensitive to inhibitors targeting this pathway, while it is less sensitive to R-CHOP treatment. In contrast, GCB-EZB-DLBCL is more sensitive to chemoimmunotherapy and dependent on tonic BCR signaling mainly engaging the PI3-kinase pathway. Other genetic subtypes such as BN2, characterized by genetic alterations in BCL6 and NOTCH2, are associated with the ABC, GCB, and unclassifiable gene expression profiles, highlighting further molecular complexity of the disease. Even though the genomic characterization of DLBCL has revolutionized our molecular understanding of DLBCL, challenges remain. About half of the DLBCL cases that fail to respond to first-line chemoimmunotherapy cannot be diagnosed and explained by mere genomics. Thus, more efficient diagnostic and therapeutic strategies for such high-risk DLBCLs are urgently needed. There is growing evidence that tumor cell-intrinsic mechanisms beyond genomics, such as deregulated post-transcriptional and translational programs, as well as tumor microenvironment (TME)-related features strongly contribute to the pathophysiology of DLBCL and thereby critically influence the therapeutic responsiveness of the individual tumors [3].

Accordingly, there is the need to provide suitable biomarkers, particularly predictive biomarkers, for diagnosing high-risk DLBCL in a patient. Furthermore, there is the need for a method that allows for the diagnosis, prognosis, and/or stratification of a diffuse large B-cell lymphoma (DLBCL) of a patient. There is also the need to provide effective means to identify high-risk DLBCL, particularly means allowing to provide a prognosis for a patient. Furthermore, there is the need to predict an R-CHOP treatment outcome. There is also the need for means that allow for personalized treatments of DLBCL patients.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method for the diagnosis, prognosis, and/or stratification of a diffuse large B-cell lymphoma (DLBCL) of a patient, comprising the steps of
a) providing a sample of said patient,
b) determining whether said sample comprises at least two, preferably at least three, more preferably at least four, even more preferably all of features i)-v):
   i) a genotype comprising
      an activated B-cell genotype with a MYD88 L265P mutation and a CD79B mutation, or
      a double-hit signature positive germinal center B-cell genotype;
      wherein, preferably, said genotype further comprises a BTG1 mutation and/or a TBL1XR1 mutation;
   ii) a dark-zone phenotype;
   iii) an enhanced signaling of TCF3, TCF4, MYC, BCR, MYD88, and/or PI3K compared to a control; wherein, preferably, the control is a reference sample, a collection of reference samples, a reference value, or a collection of reference values; wherein, more preferably, the control is a reference sample, a collection of reference samples, a reference value, or a collection of reference values of a healthy individual, a healthy cohort, or a cohort of DLBCL patients;
   iv) an enhanced expression of one or more, preferably a plurality of proteins selected from ribosomal proteins and ribosome-associated proteins, preferably ribosomal proteins, compared to the control;
   v) a tumor microenvironment characterized by depleted T cells compared to the control, preferably by depleted CD8+ effector memory T-cells compared to the control;
   wherein the presence of at least two, preferably at least three, more preferably at least four, even more preferably all of features i)-v) in said sample of said patient is indicative for the presence of a high-risk DLBCL in said patient.

In one embodiment, said determining in step b) comprises determining whether said sample comprises an enhanced signaling of TCF3, TCF4, MYC, BCR, MYD88, and/or PI3K compared to the control; wherein, preferably, said determining comprises determining whether said sample comprises an enhanced signaling of at least two, preferably at least three, more preferably at least four, even more preferably all of TCF3, TCF4, MYC, BCR, MYD88, and PI3K compared to the control.

In one embodiment, said step b) comprises determining whether said sample comprises at least two, preferably at least three, more preferably at least four, even more preferably all of features i)-v):
i) a genotype comprising
   an activated B-cell genotype with a MYD88 L265P mutation and a CD79B mutation;
   wherein, preferably, said genotype further comprises a BTG1 mutation and/or a TBL1XR1 mutation;
ii) a dark-zone phenotype;
iii) an enhanced signaling of TCF4, MYC, BCR, MYD88, and/or PI3K compared to the control, preferably of TCF4, MYC, BCR, MYD88, and PI3K compared to the control;
iv) an enhanced expression of one or more, preferably a plurality of proteins selected from ribosomal proteins and ribosome-associated proteins, preferably ribosomal proteins, compared to the control;
v) a tumor microenvironment characterized by depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk DLBCL in said patient.

In one embodiment, said step b) comprises determining whether said sample comprises at least two, preferably at least three, more preferably at least four, even more preferably all of features i)-v):
i) a genotype comprising
   a double-hit signature positive germinal center B-cell genotype;
   wherein, preferably, said genotype further comprises a BTG1 mutation and/or a TBL1XR1 mutation;
ii) a dark-zone phenotype;
iii) an enhanced signaling of TCF3, MYC, BCR, and/or PI3K compared to the control, preferably of TCF3, MYC, BCR, and PI3K compared to the control;
iv) an enhanced expression of one or more, preferably a plurality of proteins selected from ribosomal proteins and ribosome-associated proteins, preferably ribosomal proteins, compared to the control;
v) a tumor microenvironment characterized by depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk DLBCL in said patient.

In one embodiment, said determining in step b) comprises determining whether said sample comprises an enhanced expression of one or more, preferably a plurality of proteins selected from ribosomal proteins and ribosome-associated proteins, preferably ribosomal proteins, compared to the control.

In one embodiment, said determining in step b) comprises determining whether said sample comprises a tumor microenvironment characterized by depleted T cells compared to the control, preferably by depleted CD8+ effector memory T-cells compared to the control.

In one embodiment, said determining in step b) comprises determining whether said sample comprises a dark-zone phenotype; wherein, optionally, said determining in step b) comprises determining whether said sample comprises
ii) a dark-zone phenotype;
iii) an enhanced signaling of TCF3, TCF4, and/or MYC compared to the control, preferably TCF3, TCF4, and MYC compared to the control;
iv) an enhanced expression of one or more, preferably a plurality of proteins selected from ribosomal proteins and ribosome-associated proteins, preferably ribosomal proteins, compared to the control; and
v) a tumor microenvironment characterized by depleted T cells compared to the control, preferably by depleted CD8+ effector memory T-cells compared to the control.

In one embodiment, said enhanced signaling of TCF3, TCF4, MYC, BCR, MYD88, and/or PI3K compared to the control comprises an enhanced signaling of TCF4, MYC, BCR, and MYD88; TCF3, MYC, BCR, and PI3K; TCF3; TCF4; MYC; BCR; MYD88; PI3K; TCF3 and TCF4; TCF3 and MYC; TCF3 and BCR; TCF3 and MYD88; TCF3 and PI3K; TCF4 and MYC; TCF4 and BCR; TCF4 and MYD88; TCF4 and PI3K; MYC and BCR; MYC and MYD88; MYC and PI3K; BCR and MYD88; BCR and PI3K; MYD88 and PI3K; TCF3, TCF4, and MYC; TCF3, TCF4, and BCR; TCF3, TCF4, and MYD88; TCF3, TCF4, and PI3K; TCF3, MYC, and BCR; TCF3, MYC, and MYD88; TCF3, MYC, and PI3K; TCF3, BCR, and MYD88; TCF3, BCR, and PI3K; TCF3, MYD88, and PI3K; TCF4, MYC, and BCR; TCF4, MYC, and MYD88; TCF4, MYC, and PI3K; TCF4, BCR, and MYD88; TCF4, BCR, and PI3K; TCF4, MYD88, and PI3K; MYC, BCR, and MYD88; MYC, BCR, and PI3K; MYC, MYD88, and PI3K; BCR, MYD88, and PI3K; TCF3, TCF4, MYC, and BCR; TCF3, TCF4, MYC, and MYD88; TCF3, TCF4, MYC, and PI3K; TCF3, TCF4, BCR, and MYD88; TCF3, TCF4, BCR, and PI3K; TCF3, TCF4, MYD88, and PI3K; TCF3, MYC, BCR, and MYD88; TCF3, MYC, MYD88, and PI3K; TCF3, BCR, MYD88, and PI3K; TCF4, MYC, BCR, and PI3K; TCF4, MYC, MYD88, and PI3K; TCF4, BCR, MYD88, and PI3K; MYC, BCR, MYD88, and PI3K; TCF3, TCF4, MYC, BCR, and MYD88; TCF3, TCF4, MYC, BCR, and PI3K; TCF3, TCF4, MYC, MYD88, and PI3K; TCF3, TCF4, BCR, MYD88, and PI3K; TCF3, MYC, BCR, MYD88, and PI3K; TCF4, MYC, BCR, MYD88, and PI3K; or TCF3, TCF4, MYC, BCR, MYD88, and PI3K; preferably comprises an enhanced signaling of TCF4, MYC, BCR, and MYD88 or an enhanced signaling of TCF3, MYC, BCR, and PI3K; wherein said enhanced signaling is indicative for the presence of a high-risk DLBCL in said patient.

In one embodiment, said one or more, preferably a plurality of proteins are selected from PCNA, MCM6, NCL, PAICS, MYBBP1A, SSRP1, MCM4, CSDE1, MCM5, MCM3, PABPC4, SUPT16H, NASP, DDX21, BZW2, NAT10, PABPC1, RRP12, IGF2BP3, SMC4, LRPPRC, NOP2, POLD1, MTHFD1L, MKI67, FEN1, TOP2A, DDX18, PDCD11, PA2G4, SHMT2, CDC2, CCDC58, MSH2, NME1-NME2, HSPD1, GART, HSPA9, UBAP2, TOP1, MCM7, RRM1, RPL4, LYAR, GEMIN5, RRM2, GARS, IPO5, RUVBL1, GRPEL1, EEF2, NCAPH, RANBP1, IARS, RSL1D1, EEF1B2, SLIRP, NME1, ABCF2, PNPT1, MCM2, FKBP4, PBK, IMPDH2, ATXN10, DUT, TRAP1, RPL3, NOC2L, LDHA, MRTO4, YBX1, FARSB, SLC25A5, HNRNPA1, NUDC, HSP90AB1, KPNA2, ASNS, BAG2, NPM1, UTP18, TDP2, HSPE1, UTP14A, RPL35A, MARS, LDHB, RPS9, NOLC1, BTK, IMP4, NOL7, WDR3, PSMG1, SLC25A19, ALDH18A1, NIP7, CAD, C1QBP, NOL6, WDHD1, EEF1E1, NOB1, BTF3, MAD2L1, CYCS, PNO1, RPL26, TBRG4, WDR75, CACYBP, and combinations thereof.

In one embodiment, said tumor microenvironment is characterized by depleted CD4+ T cells and/or depleted CD8+ T cells compared to the control; preferably by depleted follicular helper T cells and/or memory T cells compared to the control; more preferably by depleted CD8+ effector memory T-cells compared to the control.

In one embodiment, said tumor microenvironment is further characterized by exhausted T cells compared to the control, preferably exhausted cytotoxic T cells, more preferably exhausted cytotoxic T cells of the EM2 and/or EM3 compartment.

In one embodiment, said method comprises predicting a reduced time of progression-free survival and/or a reduced time of overall survival of said patient if said sample comprises at least two, preferably at least three, more preferably at least four, even more preferably all of features i)-v).

In one embodiment, said method comprises stratifying said patient for an R-CHOP treatment, wherein said method comprises deciding in favor of or against said treatment based on the presence or absence of features i)-v); wherein, if features i)-v) are absent in said sample, the decision is in favor of the R-CHOP treatment; and wherein, if features i)-v) are present in said sample, the decision is against said R-CHOP treatment and optionally in favor of a treatment other than an R-CHOP treatment, such as a CAR T-cell therapy, a treatment with a bispecific antibody, and/or a treatment with venetoclax, ibrutinib, prednisone, obinutuzumab, and lenalidomide (ViPOR).

In one embodiment, said determining in step b) comprises analyzing
i) said genotype using sequencing such as RNA sequencing, optionally single cell sequencing;
ii) said dark-zone phenotype using RNA sequencing;
iii) said enhanced signaling using RNA sequencing, mass spectrometry, single cell sequencing, or an immunoassay such as ELISA; preferably using RNA sequencing, mass spectrometry, or single cell sequencing;
iv) said one or more, preferably said plurality of proteins selected from ribosomal proteins and ribosome-associated proteins, preferably ribosomal proteins, using RNA sequencing, mass spectrometry, or an immunoassay such as ELISA; preferably using mass spectrometry; and
v) said tumor microenvironment using flow cytometry, mass cytometry, and/or single cell sequencing;
thereby obtaining biological data, wherein, optionally, said determining further comprises analyzing said biological data using a computer-implemented method which comprises a machine learning algorithm.

In a further aspect, the present invention relates to a kit for performing a method as defined herein, the kit comprising means for determining at least two, preferably at least three, more preferably at least four, even more preferably all of features i)-v), and comprising instructions for performing the method as defined herein; wherein, preferably, the kit comprises means for performing proteomics, transcriptomics, single cell sequencing, and/or an immunoassay; wherein, more preferably, the kit comprises means for performing RNA extraction, cDNA library preparation, and/or sequencing, and optionally further comprises instructions for data analysis.

### DETAILED DESCRIPTION

The present invention relates to a method for the diagnosis, prognosis, and/or stratification of a DLBCL of a patient, comprising the steps of
a) providing a sample of said patient,
b) determining whether said sample comprises at least two, preferably at least three, more preferably at least four, even more preferably all of features i)-v):
   i) a genotype comprising
      an activated B-cell genotype with a MYD88 L265P mutation and a CD79B mutation, or
      a double-hit signature positive germinal center B-cell genotype;
      wherein, preferably, said genotype further comprises a BTG1 mutation and/or a TBL1XR1 mutation;
   ii) a dark-zone phenotype;
   iii) an enhanced signaling of TCF3, TCF4, MYC, BCR, MYD88, and/or PI3K compared to a control; wherein, preferably, the control is a reference sample, a collection of reference samples, a reference value, or a collection of reference values; wherein, more preferably, the control is a reference sample, a collection of reference samples, a reference value, or a collection of reference values of a healthy individual, a healthy cohort, or a cohort of DLBCL patients;
   iv) an enhanced expression of one or more, preferably a plurality of proteins selected from ribosomal proteins and ribosome-associated proteins, preferably ribosomal proteins, compared to the control;
   v) a tumor microenvironment characterized by depleted T cells compared to the control, preferably by depleted CD8+ effector memory T-cells compared to the control;
   wherein the presence of at least two, preferably at least three, more preferably at least four, even more preferably all of features i)-v) in said sample of said patient is indicative for the presence of a high-risk DLBCL in said patient. In a preferred embodiment, the method for the diagnosis, prognosis, and/or stratification of a DLBCL of a patient is an in vitro method and/or ex vivo method.

The advantages of the method of the invention include the ability to accurately diagnose and stratify patients with DLBCL, which allows to personalize therapy and to provide more effective treatment strategies. Thus, patient outcomes may be improved by predicting whether an R-CHOP treatment is likely or unlikely to be effective in a patient, and accordingly deciding which (possibly alternative) treatment the patient is subjected to. The inventors have identified a patient subgroup, particularly patients having a proteogenotype 4 (PG4), characterized by at least two, preferably at least three, more preferably at least four, even more preferably all of features i)-v), said features being as defined herein. The inventors have found that the respective patient subgroup (PG4), which is defined by at least two, preferably at least three, more preferably at least four, even more preferably all of features i)-v), is a high-risk DLBCL subgroup with reduced overall survival and reduced progression-free survival. Furthermore, the inventors have found that the respective patient subgroup (PG4), which is defined by at least two, preferably at least three, more preferably at least four, even more preferably all of said features i)-v), has a poor response to an R-CHOP treatment.

In one embodiment, the sample comprises lymph node tissue, spleen tissue, bone marrow tissue, tonsil tissue, blood, soft tissue, or gastrointestinal tissue; wherein said sample preferably comprises lymph node tissue, spleen tissue, bone marrow tissue, or blood; more preferably lymph node tissue. In one embodiment, the sample comprises lymphoid tissue, such as lymph node tissue, spleen tissue, bone marrow tissue, and/or tonsil tissue. Soft tissue refers to the tissues in the body that connect, support, and/or surround other structures and organs.

In one embodiment, said determining in step b) comprises determining whether said sample comprises features i) and ii); i) and iii); i) and iv); i) and v); ii) and iii); ii) and iv); ii) and v); iii) and iv); iii) and v); iv) and v); i), ii) and iii); i), ii) and iv); i), ii) and v); i), iii) and iv); i), iii) and v); i), iv) and v); ii), iii) and iv); ii), iii) and v); ii), iv) and v); iii), iv) and v); i), ii), iii) and iv); i), ii), iii) and v); i), ii), iv) and v); i), iii), iv) and v); ii), iii), iv) and v); or i), ii), iii), iv) and v);
wherein the presence of said features i) and ii); i) and iii); i) and iv); i) and v); ii) and iii); ii) and iv); ii) and v); iii) and iv); iii) and v); iv) and v); i), ii) and iii); i), ii) and iv); i), ii) and v); i), iii) and iv); i), iii) and v); i), iv) and v); ii), iii) and iv); ii), iii) and v); ii), iv) and v); iii), iv) and v); i), ii), iii) and iv); i), ii), iii) and v); i), ii), iv) and v); i), iii), iv) and v); ii), iii), iv) and v); or i), ii), iii), iv) and v) in said sample of said patient is indicative for the presence of a high-risk DLBCL in said patient. In one embodiment, the presence of said features in said sample of said patient is indicative for the presence of a DLBCL associated with a reduced overall survival and/or reduced progression-free survival of said patient.

In the context of DLBCL, the term "high-risk" typically refers to a subset of patients who exhibit poor overall and/or progression-free survival, particularly reduced overall survival and/or reduced progression-free survival compared to other subsets of DLBCL patients. High-risk DLBCL may be associated with a poor response to standard R-CHOP therapy and may require alternative therapeutic strategies.

In one embodiment, said determining in step b) comprises determining whether said sample comprises a genotype comprising an activated B-cell genotype with a MYD88 L265P mutation and a CD79B mutation, or comprising a double-hit signature positive germinal center B-cell genotype; wherein, preferably, said genotype further comprises a BTG1 mutation and/or a TBL1XR1 mutation. In a preferred embodiment, the presence of said genotype in said sample of said patient is indicative for the presence of a high-risk DLBCL in said patient.

The double-hit signature positive germinal center B-cell genotype is typically characterized by the presence of genetic alterations of the MYC gene along with either the BCL2 or BCL6 genes. In one embodiment, the activated B-cell genotype with a MYD88 L265P mutation and a CD79B mutation further comprises a BTG1 mutation and/or a TBL1XR1 mutation. In one embodiment, the double-hit signature positive germinal center B-cell genotype comprises a BTG1 mutation and/or a TBL1XR1 mutation. In one embodiment, the double-hit signature positive germinal center B-cell genotype comprises a MYC mutation and a BCL2 or BCL6 mutation, and optionally further comprises a BTG1 mutation and/or a TBL1XR1 mutation. In one embodiment, the double-hit signature positive germinal center B-cell genotype is a germinal center B-cell genotype encoding a double-hit signature, particularly encoding a double-hit gene expression signature.

In one embodiment, said genotype further comprises a BTG1 mutation and/or a TBL1XR1 mutation. The mutations may be, for example, missense mutations, nonsense mutations, frameshift mutations, and/or deletions. In one embodiment, said determining in step b) comprises determining whether said sample comprises a BTG1 mutation and/or a TBL1XR1 mutation. In a preferred embodiment, a presence of a BTG1 mutation and/or a TBL1XR1 mutation in said sample of said patient is indicative for the presence of a high-risk DLBCL in said patient.

In one embodiment, said determining in step b) comprises determining whether said sample comprises a dark-zone phenotype. In the context of DLBCL, the term "dark-zone phenotype" typically refers to a phenotype present in said sample, particularly a phenotype of lymphoma cells present in said sample, that corresponds to the phenotype of B cells found in the dark-zone of germinal centers within lymphoid tissues, for example to a transcriptome of the lymphoma cells that corresponds to the transcriptome of B cells found in the dark-zone of germinal centers within lymphoid tissues. For example, the dark-zone phenotype may comprise a high proliferation rate, a specific gene expression profile, morphological features, and/or functional characteristics similar to those of centroblasts. For example, lymphoma cells with a dark-zone phenotype may be highly proliferative, similar to the rapidly dividing centroblasts in the dark-zone. For example, lymphoma cells with a dark-zone phenotype may express genes associated with proliferation and somatic hypermutation. For example, under microscopic examination, lymphoma cells may appear similar to centroblasts, with large nuclei and prominent nucleoli. For example, the lymphoma cells may comprise functional characteristics comprising enhanced signaling pathways that are active in dark-zone B cells, such as those involving MYC, BCR, and other proliferation-related pathways. In a preferred embodiment, the presence of said dark-zone phenotype in said sample of said patient is indicative for the presence of a high-risk DLBCL in said patient. In one embodiment, the dark-zone phenotype is characterized by a centroblast gene expression signature, a GCB8/GCB10 gene expression signature, and a DHIT+ gene expression signature, said signatures preferably being as described in [1]; optionally is characterized by MYC activity.

In one embodiment, said determining in step b) comprises determining whether said sample comprises an enhanced signaling of TCF3, TCF4, MYC, BCR, MYD88, and/or PI3K compared to the control. In a preferred embodiment, the presence of said an enhanced signaling of TCF3, TCF4, MYC, BCR, MYD88, and/or PI3K in said sample of said patient is indicative for the presence of a high-risk DLBCL in said patient. In one embodiment, said determining in step b) comprises determining whether said sample comprises an enhanced signaling of at least two, preferably at least three, more preferably at least four, even more preferably all of TCF3, TCF4, MYC, BCR, MYD88, and PI3K compared to the control. In a preferred embodiment, the presence of said enhanced signaling of said at least two, preferably said at least three, more preferably said at least four, even more preferably said all of TCF3, TCF4, MYC, BCR, MYD88, and PI3K in said sample of said patient is indicative for the presence of a high-risk DLBCL in said patient. In one embodiment, said enhanced signaling comprises an enhanced gene expression of target genes of said TCF3, TCF4, MYC, BCR, MYD88, and PI3K, respectively. In one embodiment, said enhanced signaling is determined by analyzing gene expression of target genes of said TCF3, TCF4, MYC, BCR, MYD88, and PI3K, respectively.

In one embodiment, said determining in step b) comprises determining whether said sample comprises enhanced signaling of TCF4, MYC, BCR, and MYD88; TCF3, MYC, BCR, and PI3K; TCF3; TCF4; MYC; BCR; MYD88; PI3K; TCF3 and TCF4; TCF3 and MYC; TCF3 and BCR; TCF3 and MYD88; TCF3 and PI3K; TCF4 and MYC; TCF4 and BCR; TCF4 and MYD88; TCF4 and PI3K; MYC and BCR; MYC and MYD88; MYC and PI3K; BCR and MYD88; BCR and PI3K; MYD88 and PI3K; TCF3, TCF4, and MYC; TCF3, TCF4, and BCR; TCF3, TCF4, and MYD88; TCF3, TCF4, and PI3K; TCF3, MYC, and BCR; TCF3, MYC, and MYD88; TCF3, MYC, and PI3K; TCF3, BCR, and MYD88; TCF3, BCR, and PI3K; TCF3, MYD88, and PI3K; TCF4, MYC, and BCR; TCF4, MYC, and MYD88; TCF4, MYC, and PI3K; TCF4, BCR, and MYD88; TCF4, BCR, and PI3K; TCF4, MYD88, and PI3K; MYC, BCR, and MYD88; MYC, BCR, and PI3K; MYC, MYD88, and PI3K; BCR, MYD88, and PI3K; TCF3, TCF4, MYC, and BCR; TCF3, TCF4, MYC, and MYD88; TCF3, TCF4, MYC, and PI3K; TCF3, TCF4, BCR, and MYD88; TCF3, TCF4, BCR, and PI3K; TCF3, TCF4, MYD88, and PI3K; TCF3, MYC, BCR, and MYD88; TCF3, MYC, MYD88, and PI3K; TCF3, BCR, MYD88, and PI3K; TCF4, MYC, BCR, and PI3K; TCF4, MYC, MYD88, and PI3K; TCF4, BCR, MYD88, and PI3K; MYC, BCR, MYD88, and PI3K; TCF3, TCF4, MYC, BCR, and MYD88; TCF3, TCF4, MYC, BCR, and PI3K; TCF3, TCF4, MYC, MYD88, and PI3K; TCF3, TCF4, BCR, MYD88, and PI3K; TCF3, MYC, BCR, MYD88, and PI3K; TCF4, MYC, BCR, MYD88, and PI3K; or TCF3, TCF4, MYC, BCR, MYD88, and PI3K; preferably determining whether said sample comprises an enhanced signaling of TCF4, MYC, BCR, and MYD88 or an enhanced signaling of TCF3, MYC, BCR, and PI3K; wherein said enhanced signaling is indicative for the presence of a high-risk DLBCL in said patient.

In one embodiment, an enhanced signaling of TCF4, MYC, BCR, and MYD88; TCF3, MYC, BCR, and PI3K; TCF3; TCF4; MYC; BCR; MYD88; PI3K; TCF3 and TCF4; TCF3 and MYC; TCF3 and BCR; TCF3 and MYD88; TCF3 and PI3K; TCF4 and MYC; TCF4 and BCR; TCF4 and MYD88; TCF4 and PI3K; MYC and BCR; MYC and MYD88; MYC and PI3K; BCR and MYD88; BCR and PI3K; MYD88 and PI3K; TCF3, TCF4, and MYC; TCF3, TCF4, and BCR; TCF3, TCF4, and MYD88; TCF3, TCF4, and PI3K; TCF3, MYC, and BCR; TCF3, MYC, and MYD88; TCF3, MYC, and PI3K; TCF3, BCR, and MYD88; TCF3, BCR, and PI3K; TCF3, MYD88, and PI3K; TCF4, MYC, and BCR; TCF4, MYC, and MYD88; TCF4, MYC, and PI3K; TCF4, BCR, and MYD88; TCF4, BCR, and PI3K; TCF4, MYD88, and PI3K; MYC, BCR, and MYD88; MYC, BCR, and PI3K; MYC, MYD88, and PI3K; BCR, MYD88, and PI3K; TCF3, TCF4, MYC, and BCR; TCF3, TCF4, MYC, and MYD88; TCF3, TCF4, MYC, and PI3K; TCF3, TCF4, BCR, and MYD88; TCF3, TCF4, BCR, and PI3K; TCF3, TCF4, MYD88, and PI3K; TCF3, MYC, BCR, and MYD88; TCF3, MYC, MYD88, and PI3K; TCF3, BCR, MYD88, and PI3K; TCF4, MYC, BCR, and PI3K; TCF4, MYC, MYD88, and PI3K; TCF4, BCR, MYD88, and PI3K; MYC, BCR, MYD88, and PI3K; TCF3, TCF4, MYC, BCR, and MYD88; TCF3, TCF4, MYC, BCR, and PI3K; TCF3, TCF4, MYC, MYD88, and PI3K; TCF3, TCF4, BCR, MYD88, and PI3K; TCF3, MYC, BCR, MYD88, and PI3K; TCF4, MYC, BCR, MYD88, and PI3K; or TCF3, TCF4, MYC, BCR, MYD88, and PI3K; preferably of TCF4, MYC, BCR, and MYD88, or of TCF3, MYC, BCR, and PI3K; is indicative for the presence of a high-risk DLBCL in said patient. In one embodiment, said enhanced signaling is an enhanced signaling of MYC, BCR and PI3K; and said genotype is either an activated B-cell genotype with a MYD88 L265P mutation and a CD79B mutation, or a double-hit signature positive germinal center B-cell genotype. In one embodiment, said enhanced signaling is an enhanced signaling of TCF4, MYC, BCR, MYD88, and PI3K; and said genotype is an activated B-cell genotype with a MYD88 L265P mutation and a CD79B mutation. In one embodiment, said enhanced signaling is an enhanced signaling of TCF3, MYC, BCR, and PI3K; and said genotype is a double-hit signature positive germinal center B-cell genotype.

In one embodiment, the control is a reference sample, a collection of reference samples, a reference value, or a collection of reference values. In a preferred embodiment, the control is a reference sample, a collection of reference samples, a reference value, or a collection of reference values of a healthy individual, a healthy cohort, or a cohort of DLBCL patients. For example, the control may be a collection of reference samples or a collection of reference values of a plurality of DLBCL patients, wherein said plurality of DLBCL patients comprises DLBCL patients other than high-risk DLBCL patients. For example, the control may be a reference value which indicates an average value found in healthy individuals or in DLBCL patients, wherein said DLBCL patients comprise DLBCL patients other than high-risk DLBCL patients.

In one embodiment, said determining in step b) comprises determining whether said sample comprises an enhanced expression of one or more, preferably a plurality of proteins selected from ribosomal proteins and ribosome-associated proteins, preferably ribosomal proteins, compared to the control. In a preferred embodiment, the proteins selected from ribosomal proteins and ribosome-associated proteins are selected from PCNA, MCM6, NCL, PAICS, MYBBP1A, SSRP1, MCM4, CSDE1, MCM5, MCM3, PABPC4, SUPT16H, NASP, DDX21, BZW2, NAT10, PABPC1, RRP12, IGF2BP3, SMC4, LRPPRC, NOP2, POLD1, MTHFD1L, MKI67, FEN1, TOP2A, DDX18, PDCD11, PA2G4, SHMT2, CDC2, CCDC58, MSH2, NME1-NME2, HSPD1, GART, HSPA9, UBAP2, TOP1, MCM7, RRM1, RPL4, LYAR, GEMIN5, RRM2, GARS, IPO5, RUVBL1, GRPEL1, EEF2, NCAPH, RANBP1, IARS, RSL1D1, EEF1B2, SLIRP, NME1, ABCF2, PNPT1, MCM2, FKBP4, PBK, IMPDH2, ATXN10, DUT, TRAP1, RPL3, NOC2L, LDHA, MRTO4, YBX1, FARSB, SLC25A5, HNRNPA1, NUDC, HSP90AB1, KPNA2, ASNS, BAG2, NPM1, UTP18, TDP2, HSPE1, UTP14A, RPL35A, MARS, LDHB, RPS9, NOLC1, BTK, IMP4, NOL7, WDR3, PSMG1, SLC25A19, ALDH18A1, NIP7, CAD, C1QBP, NOL6, WDHD1, EEF1E1, NOB1, BTF3, MAD2L1, CYCS, PNO1, RPL26, TBRG4, WDR75, CACYBP, and combinations thereof. In one embodiment, said ribosomal proteins are selected from RPL4, RPL3, RPL35A, RPS9, and RPL26. In one embodiment, said ribosome-associated proteins are selected from MCM6, NCL, PAICS, MYBBP1A, SSRP1, MCM4, CSDE1, MCM5, MCM3, PABPC4, SUPT16H, NASP, DDX21, BZW2, NAT10, PABPC1, RRP12, IGF2BP3, SMC4, LRPPRC, NOP2, POLD1, MTHFD1L, MKI67, FEN1, TOP2A, DDX18, PDCD11, PA2G4, SHMT2, CDC2, CCDC58, MSH2, NME1-NME2, HSPD1, GART, HSPA9, UBAP2, TOP1, MCM7, RRM1, LYAR, GEMIN5, RRM2, GARS, IPO5, RUVBL1, GRPEL1, EEF2, NCAPH, RANBP1, IARS, RSL1D1, EEF1B2, SLIRP, NME1, ABCF2, PNPT1, MCM2, FKBP4, PBK, IMPDH2, ATXN10, DUT, TRAP1, NOC2L, LDHA, MRTO4, YBX1, FARSB, SLC25A5, HNRNPA1, NUDC, HSP90AB1, KPNA2, ASNS, BAG2, NPM1, UTP18, TDP2, HSPE1, UTP14A, MARS, LDHB, NOLC1, BTK, IMP4, NOL7, WDR3, PSMG1, SLC25A19, ALDH18A1, NIP7, CAD, C1QBP, NOL6, WDHD1, EEF1E1, NOB1, BTF3, MAD2L1, CYCS, PNO1, TBRG4, WDR75, and CACYBP. Optionally, said ribosome-associated proteins are selected from NCL, DDX21, RRP12, NOP2, PDCD11, PA2G4, MRTO4, NOLC1, IMP4, NOL7, NIP7, NOL6, and PNO1. In one embodiment, ribosome-associated proteins are proteins that interact with ribosomes, such as proteins involved in ribosome biogenesis, ribosome assembly, ribosome function, and/or ribosome regulation. In one embodiment, ribosome-associated proteins are selected from ribosome assembly factor proteins, ribosomal RNA processing proteins, initiation factor proteins, elongation factor proteins, termination factor proteins, ribosome recycling factor proteins, ribosome surveillance factor proteins, and combinations thereof.

In one embodiment, said determining in step b) comprises determining whether said sample comprises a tumor microenvironment characterized by depleted T cells compared to the control, preferably by depleted CD8+ effector memory T-cells compared to the control. In one embodiment, said depleted T cells comprise depleted CD4+ T cells and/or depleted CD8+ T cells. In one embodiment, said depleted T cells comprise depleted follicular helper T cells and/or memory T cells, such as depleted CD8+ effector memory T-cells. In one embodiment, said tumor microenvironment is characterized by depleted CD4+ T cells and/or depleted CD8+ T cells compared to the control; preferably by depleted follicular helper T cells and/or memory T cells compared to the control; more preferably by depleted CD8+ effector memory T-cells compared to the control. The term "depleted T cells", as used herein, refers to a reduced number or proportion of T cells, preferably a reduced number of T cells, in a sample compared to a control. Depleted T cells may lead to a weakened immune response, making the body more susceptible to infections and reducing its ability to fight cancer. In one embodiment, said tumor microenvironment is further characterized by exhausted T cells compared to the control, preferably exhausted cytotoxic T cells, more preferably exhausted cytotoxic T cells of the EM2 and/or EM3 compartment. For example, the exhausted T cells may be exhausted cytotoxic T cells and/or exhausted T cells of the EM2 and/or EM3 compartment. The term "exhausted T cells", as used herein, relates to dysfunctional T cells, e.g. T cells which do not effectively respond to antigens. For example, exhausted T cells may have been persistently exposed to antigens and, as a result, lose their ability to effectively respond to these antigens. Exhausted T cells may have a reduced effector function, an increased expression of inhibitory receptors which negatively regulate T cell activity, an altered metabolic state, and/or epigenetic changes. The state of exhaustion typically limits the ability of T cells to control infections and tumors effectively. In one embodiment, the CD8+ effector memory T-cells are CD8+ effector memory type 2 compartment T-cells. The presence of exhausted T cells may be determined, for example, using the gene expression signature described in [2].

In one embodiment, the presence of at least two, preferably at least three, more preferably at least four, even more preferably all of features i)-v) in said sample of said patient is indicative for the presence of a high-risk DLBCL in said patient. For example, a sample comprising said genotype, said dark-zone phenotype, said enhanced signaling of at least one of TCF3, TCF4, MYC, BCR, MYD88, and PI3K, said enhanced expression of at least one protein selected from ribosomal proteins and ribosome-associated proteins, and said tumor microenvironment is indicative for the presence of a high-risk DLBCL in said patient, particularly is indicative for a predicted R-CHOP treatment outcome being poor. In one embodiment, the presence of features i) and ii); i) and iii); i) and iv); i) and v); ii) and iii); ii) and iv); ii) and v); iii) and iv); iii) and v); iv) and v); i), ii) and iii); i), ii) and iv); i), ii) and v); i), iii) and iv); i), iii) and v); i), iv) and v); ii), iii) and iv); ii), iii) and v); ii), iv) and v); iii), iv) and v); i), ii), iii) and iv); i), ii), iii) and v); i), ii), iv) and v); i), iii), iv) and v); ii), iii), iv) and v); or i), ii), iii), iv) and v) in said sample of said patient is indicative for the presence of a high-risk DLBCL in said patient, particularly is indicative for the presence of a DLBCL associated with a reduced overall survival and/or reduced progression-free survival of said patient. In one embodiment, a high-risk DLBCL is DLBCL associated with a reduced overall survival and/or reduced progression-free survival. In one embodiment, a high-risk DLBCL is DLBCL associated with a poor R-CHOP treatment outcome. In one embodiment, a high-risk DLBCL is DLBCL associated with a poor prognosis for said patient. By determining all of features i)-v), the method becomes very robust and comprehensive, allowing for a highly accurate assessment of the disease state. The method of the invention allows for a precise determination of the patient's risk level, particularly if at least three, preferably at least four, more preferably all of features i)-v) are determined. Furthermore, the method of the invention allows to decide on the most suitable treatment strategy based on the presence of at least three, preferably at least four, more preferably all of features i)-v).

In one embodiment, said step b) comprises determining whether said sample comprises at least two, preferably at least three, more preferably at least four, even more preferably all of features i)-v):
i) a genotype comprising
   an activated B-cell genotype with a MYD88 L265P mutation and a CD79B mutation;
   wherein, preferably, said genotype further comprises a BTG1 mutation and/or a TBL1XR1 mutation;
ii) a dark-zone phenotype;
iii) an enhanced signaling of TCF4, MYC, BCR, MYD88, and/or PI3K compared to the control, preferably of TCF4, MYC, BCR, MYD88, and PI3K compared to the control;
iv) an enhanced expression of one or more, preferably a plurality of proteins selected from ribosomal proteins and ribosome-associated proteins, preferably ribosomal proteins, compared to the control;
v) a tumor microenvironment characterized by depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk DLBCL in said patient. In one embodiment, said step b) comprises determining whether said sample comprises an enhanced signaling of at least TCF4, BCR, and MYD88; an enhanced expression of one or more, preferably a plurality of proteins selected from ribosomal proteins and ribosome-associated proteins; and a tumor microenvironment characterized by depleted T cells. In one embodiment, the presence of an enhanced signaling of at least TCF4, BCR, and MYD88; the enhanced expression of one or more, preferably a plurality of proteins selected from ribosomal proteins and ribosome-associated proteins; and the tumor microenvironment characterized by depleted T cells in said sample of said patient is indicative for the presence of a high-risk DLBCL in said patient.

In one embodiment, said step b) comprises determining whether said sample comprises at least two, preferably at least three, more preferably at least four, even more preferably all of features i)-v):
i) a genotype comprising
   a double-hit signature positive germinal center B-cell genotype;
   wherein, preferably, said genotype further comprises a BTG1 mutation and/or a TBL1XR1 mutation;
ii) a dark-zone phenotype;
iii) an enhanced signaling of TCF3, MYC, BCR, and/or PI3K compared to the control, preferably of TCF3, MYC, BCR, and PI3K compared to the control;
iv) an enhanced expression of one or more, preferably a plurality of proteins selected from ribosomal proteins and ribosome-associated proteins, preferably ribosomal proteins, compared to the control;
v) a tumor microenvironment characterized by depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk DLBCL in said patient.

In one embodiment, said step b) comprises determining whether said sample comprises a genotype comprising a double-hit signature positive germinal center B-cell genotype; an enhanced signaling of at least TCF3 and MYC; an enhanced expression of one or more, preferably a plurality of proteins selected from ribosomal proteins and ribosome-associated proteins; and a tumor microenvironment characterized by depleted T cells. In one embodiment, the presence of a genotype comprising a double-hit signature positive germinal center B-cell genotype; an enhanced signaling of at least TCF3 and MYC; an enhanced expression of one or more, preferably a plurality of proteins selected from ribosomal proteins and ribosome-associated proteins; and a tumor microenvironment characterized by depleted T cells in said sample of said patient is indicative for the presence of a high-risk DLBCL in said patient.

In one embodiment, said determining in step b) comprises determining whether said sample comprises
ii) a dark-zone phenotype;
iii) an enhanced signaling of TCF3, TCF4, MYC, BCR, MYD88, and/or PI3K compared to a control; preferably an enhanced signaling of TCF3, TCF4, and/or MYC compared to the control; more preferably TCF3, TCF4, and MYC compared to the control;
iv) an enhanced expression of one or more, preferably a plurality of proteins selected from ribosomal proteins and ribosome-associated proteins, preferably ribosomal proteins, compared to the control; and
v) a tumor microenvironment characterized by depleted T cells compared to the control, preferably by depleted CD8+ effector memory T-cells compared to the control;
wherein the presence of features ii)-v) in said sample of said patient is indicative for the presence of a high-risk DLBCL in said patient. The inventors have found that a high-risk DLBCL is reliably identified, for example if features ii)-v) are present in said sample. The inventors have found that, for example if features ii)-v) are present in said sample, the patient has a poor prognosis. The inventors have found that, for example if features ii)-v) are present in said sample, the patient is likely to have a poor response to an R-CHOP treatment.

In one embodiment, said method comprises predicting a reduced time of progression-free survival and/or a reduced time of overall survival of said patient if said sample comprises at least two, preferably at least three, more preferably at least four, even more preferably all of features i)-v). The inventors have found that a reduced time of progression-free survival and/or a reduced time of overall survival of said patient is effectively and reliable predicted using features i)-v). Particularly, the inventors have found that a treatment outcome is reliable predicted as being poor if the sample of the patient comprises at least two, preferably at least three, more preferably at least four, even more preferably all of features i)-v). The more features are determined and present in said sample, the higher the precision of predicting a high-risk DLBCL. If all of features i)-v) are determined to be present in said sample, the confidence of the prediction is extremely high.

In one embodiment, said method comprises stratifying said patient for an R-CHOP treatment, wherein said method comprises deciding in favor of or against said treatment based on the presence or absence of features i)-v); wherein, if features i)-v) are absent in said sample, the decision is in favor of the R-CHOP treatment; and wherein, if features i)-v) are present in said sample, the decision is against said R-CHOP treatment and optionally in favor of a treatment other than an R-CHOP treatment, such as a CAR T-cell therapy, a treatment with a bispecific antibody, and/or a treatment with venetoclax, ibrutinib, prednisone, obinutuzumab, and lenalidomide (ViPOR). R-CHOP is a combination chemotherapy commonly used to treat certain types of non-Hodgkin lymphoma (NHL), including DLBCL. The acronym R-CHOP stands for the five drugs comprised by the treatment, particularly Rituximab, Cyclophosphamide, Doxorubicin, Vincristine, and Prednisone.

Advantageously, by determining whether said sample comprises at least two, preferably at least three, more preferably at least four, even more preferably all of features i)-v), it is possible to determine whether a patient suffers from or is at risk of acquiring high-risk DLBCL. Advantageously, an appropriate treatment for said patient may be selected if it is identified whether the patient has a high-risk DLBCL. For example, a patient having high-risk DLBCL with at least three, preferably at least four, more preferably all of features i)-v) may be subjected to a treatment other than an R-CHOP treatment, since the predicted treatment outcome of an R-CHOP treatment of said patient is poor. Accordingly, by using a method of the present invention, patients may be stratified for an appropriate treatment.

Stratification typically allows to categorize patients into different groups based on specific characteristics, with the goal of tailoring treatment strategies to optimize outcomes. Treatment stratification aims to ensure that patients receive the most appropriate and effective therapies based on their individual profiles. In one embodiment, stratification comprises identifying whether a treatment is suitable for a patient. Advantageously, tailoring treatments to individual patients increases the likelihood of success and reduces the risk of adverse effects. For example, by determining whether a patient is likely to respond to a treatment such as an R-CHOP treatment, treatments with predicted poor outcomes may be avoided. Advantageously, the method of the invention allows to predict whether a patient is likely to respond to an R-CHOP treatment. In one embodiment, if said sample comprises all of features i)-v), the patient is unlikely to respond to an R-CHOP treatment.

In one embodiment, said determining in step b) comprises analyzing
i) said genotype using sequencing such as RNA sequencing, optionally single cell sequencing;
ii) said dark-zone phenotype using RNA sequencing;
iii) said enhanced signaling using RNA sequencing, mass spectrometry, single cell sequencing, or an immunoassay such as ELISA; preferably using RNA sequencing, mass spectrometry, or single cell sequencing;
iv) said one or more, preferably said plurality of proteins selected from ribosomal proteins and ribosome-associated proteins, preferably ribosomal proteins, using RNA sequencing, mass spectrometry, or an immunoassay such as ELISA; preferably using mass spectrometry; and/or
v) said tumor microenvironment using flow cytometry, mass cytometry, and/or sequencing such as single cell sequencing;
thereby obtaining biological data, wherein, optionally, said determining further comprises analyzing said biological data using a computer-implemented method which comprises a machine learning algorithm. In one embodiment, the computer-implemented method comprises a classifier which involves features i)-v).

In one embodiment, said determining in step b) comprises determining whether said sample comprises
iii) an enhanced signaling of TCF3, TCF4, MYC, BCR, MYD88, and/or PI3K compared to the control;
iv) an enhanced expression of one or more, preferably a plurality of proteins selected from ribosomal proteins and ribosome-associated proteins, preferably ribosomal proteins, compared to the control; wherein the presence of said enhanced signaling and said enhanced expression in said sample of said patient is indicative for the presence of a high-risk diffuse large B-cell lymphoma (DLBCL) in said patient.

The inventors have found that a high-risk DLBCL is efficiently identified, for example if features iii)-iv) are present in said sample. The inventors have found that, for example if features iii)-iv) are present in said sample, the patient is likely to exhibit reduced overall and/or progression-free survival. The inventors have found that, for example if features iii)-iv) are present in said sample, the patient is likely to have a poor response to an R-CHOP treatment.

In a further aspect, the present invention relates to a kit for performing a method of the invention, the kit comprising means for determining at least two, preferably at least three, more preferably at least four, even more preferably all of features i)-v), and comprising instructions for performing the method of the invention; wherein, preferably, the kit comprises means for performing proteomics, transcriptomics, single cell sequencing, and/or an immunoassay; wherein, more preferably, the kit comprises means for performing RNA extraction, cDNA library preparation, and/or sequencing, and optionally further comprises instructions for data analysis. The kit may be a kit for the diagnosis, prognosis, and/or stratification of a DLBCL of a patient.

By an integrated proteogenomic approach, the inventors have further resolved the so far unexplored molecular and clinical heterogeneity of DLBCL. Advantageously, the inventors have identified predictive biomarkers for high-risk disease, particularly features i)-v). Typically, high-risk DLBCL is characterized by poor overall and progression-free survival. Features i)-v) allow to efficiently and reliably predict patient R-CHOP treatment outcome.

The present invention also relates to a method for the diagnosis, prognosis, and/or stratification of a DLBCL of a patient, comprising the step of determining whether a sample obtained from a patient comprises at least two, preferably at least three, more preferably at least four, even more preferably all of features i)-v), said features i)-v) being as defined herein, wherein the presence of at least two, preferably at least three, more preferably at least four, even more preferably all of features i)-v) in said sample of said patient is indicative for the presence of a high-risk DLBCL in said patient. In this aspect, said DLBCL, said patient, said determining, said sample, said features, and said indication are as defined herein.

In one embodiment, the term "patient" relates to a human or an animal, preferably a human. The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated. The term "about", as used herein in the context of numbers or data, intends to include deviations (±) which usually are to be considered in the respective technical field.

### BRIEF DESCRIPTION OF THE FIGURES

The present invention is now further described by reference to the following figures. All methods mentioned in the figure descriptions below were carried out as described in detail in the examples.

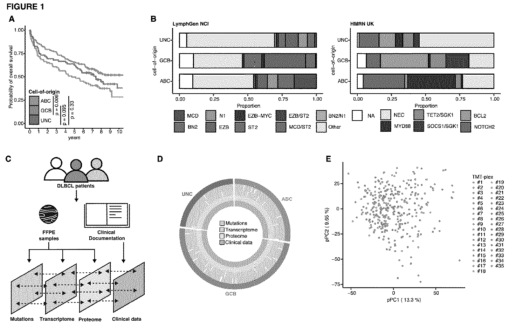

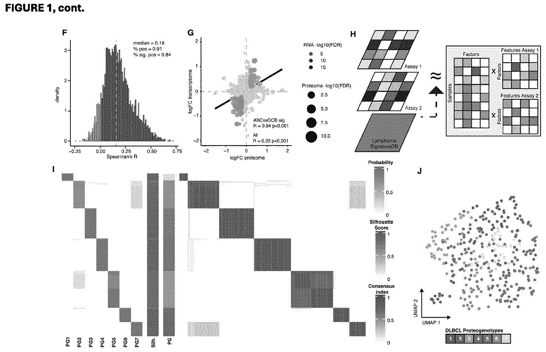

**Figure 1****: Identification of DLBCL proteogenotypes by integrated proteogenomics.** A) Overall survival estimates for the discovery cohort stratified by cell-of-origin status. B) Distribution of genetic subtypes (LymphGen, left panel) or HRMN classification-derived subtypes (right panel) per cell-of-origin status. C) Scheme of the integrated proteogenomic approach for characterization of FFPE DLBCL tissue. D) Data completeness per sample throughout all data layers. E) Probabilistic principal component analysis (pPCA) of normalized protein intensity data. Individual samples are color-coded according to their TMT labels. F) Transcriptomic to proteomic expression correlation for all detected mRNA/protein pairs. Positive correlation is highlighted in dark blue, negative correlation in grey. The grey vertical line depicts the median Spearman's R across all mRNA/protein pair correlations. G) Correlation of transcriptomic (y-axis) and proteomic (x-axis) expression data for mRNA and/or proteins differentially expressed in ABC and GCB DLBCL. Each dot represents an mRNA/protein pair color-coded for significantly increased expression in ABC cases (blue), in GCB cases (orange) and others (grey). The shade denotes the significance of the differential expression at the transcriptomic level, while the size of each dot corresponds to the significance of the differential expression at the proteomic level. Spearman's R for the correlation of all RNA/protein pairs (*All*) and for pairs significantly differentially expressed at both data layers (*ABCvsGCBsig*) are reported. H) Pictogram of the bioinformatic multi-omics integration approach *MuVI.* I) Result metrics from the graph-based consensus clustering of the inferred latent space after correction for the cell-of-origin (COO) status including the probability of iterative re-clustering into the same proteogenotypes (PG), the silhouette score and the matrix of consensus indices across all tested iterations. J) UMAP visualization of the COO-corrected multi-omic latent space with each sample color-coded according to its respective DLBCL PG inferred from graph-based consensus clustering.

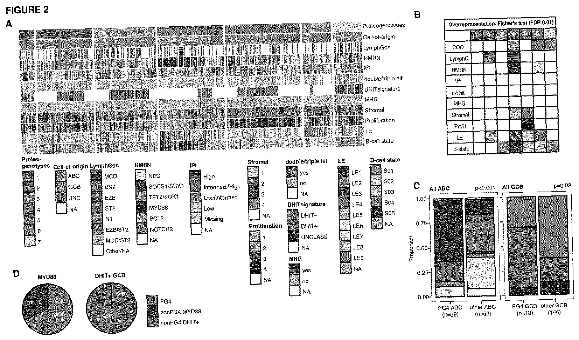

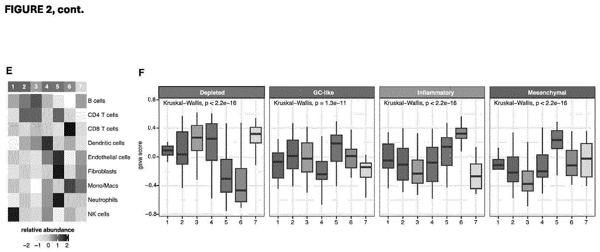

**Figure 2****: Phenotypic characteristics of DLBCL proteogenotypes.** A) Disease-related and clinical meta data for each of the identified DLBCL proteogenotypes (PG). COO, cell-of-origin; DHIT, double-hit signature; IPI, international prognostic index; LE, lymphoma ecotype; MHG, molecular high-grade subtype. B) Overrepresentation analysis by Fisher's test of annotated meta features for each proteogenomic subtype. Boxes are color-coded if a significant overrepresentation of a feature was identified with FDR of 0.01 after multiple hypothesis correction (Benjamini-Hochberg method). C) Proportion of MYD88 cases in PG4 ABC versus non-PG4 ABC cases (left panel) and DHIT signature-positive cases in PG4 GCB in comparison to non-PG4 GCB (right panel). Significance testing (for MYD88 in PG4 vs. all others and DHIT+ in PG4 vs. all others) via Fisher's test. D) Number of PG4 cases among all MYD88 cases (left side) and among all DHIT+ cases (right side) E) Relative abundance of cell-types per DLBCL PG as determined by digital deconvolution using CIBERSORT.x based on transcriptomic expression data. F) Gene-set-variation-analysis (GSVA) scores for the TME phenotypic themes derived from *Kotlov et al.* across each DLBCL PG. Testing for significant statistical differences within each theme and across all seven PGs was performed by Kruskal-Wallis test.

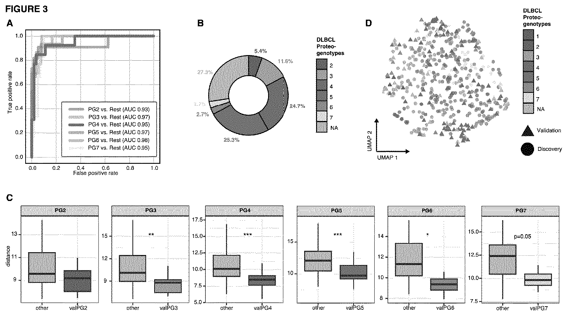

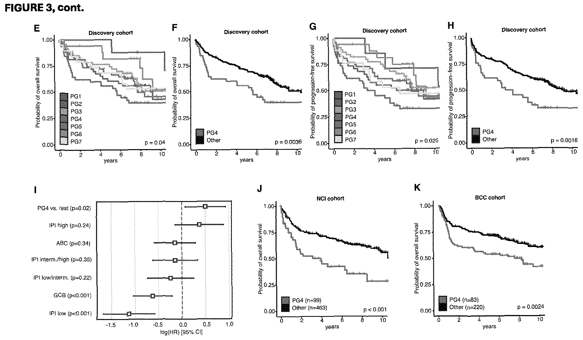

**Figure 3****: DLBCL proteogenotype validation and survival phenotypes.** A) Receiver operator characteristics (ROC) for prediction of PGs in the validation cohort with XGBOOST model trained on the discovery cohort. B) Distribution of predicted DLBCL PGs in the validation cohort. C) UMAP visualization of the joint latent space of the discovery and validation cohorts. Dots: Discovery cohort samples; triangles: Validation cohort samples. D) Distance of PG-predicted validation cohort samples to the centroid of all discovery cohort samples within the joint latent space. Statistical significance was determined by Wilcoxon-Mann-Whitney U test and multiple hypothesis correction using the Benjamini-Hochberg-Method. * p<0.05; ** p<0.01, *** p<0.001, **** p<0.0001. E) Overall survival (OS) estimates stratified by DLBCL PGs in all patients with DLBCL NOS and R-CHOP treatment. F) OS estimate stratified for PG4 versus all other DLBCL PGs in DLBCL NOS with R-CHOP treatment. G) Progression-free survival (PFS) estimates stratified by DLBCL PGs in all patients with DLBCL NOS and R-CHOP treatment. H) PFS estimate stratified by PG4 versus all other DLBCL PGs in DLBCL NOS with R-CHOP treatment. Statistical testing was performed by log-rank test in E-H. I) Multivariate cox-regression testing for PG4 vs. other DLBCL PGs including IPI and cell-of-origin status as covariates in DLBCL NOS with R-CHOP treatment. J) OS estimates for predicted PG4 versus all other PGs in the NCI cohort. K) OS estimates for predicted PG4 versus all other PGs in the BCC cohort.

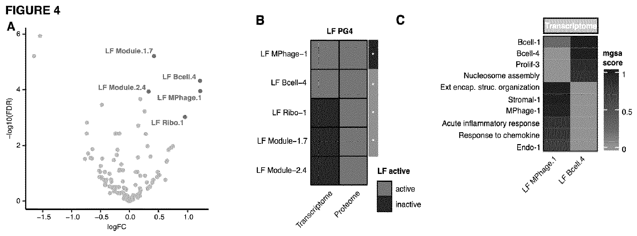

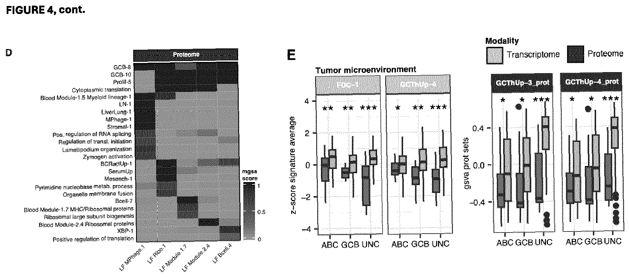

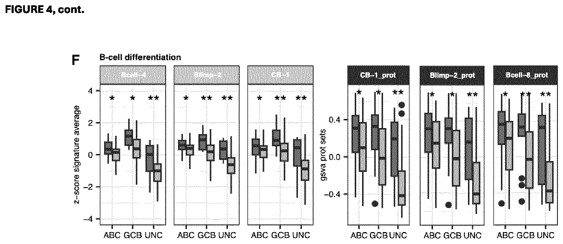

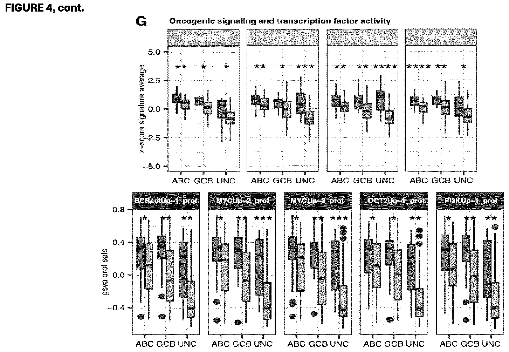

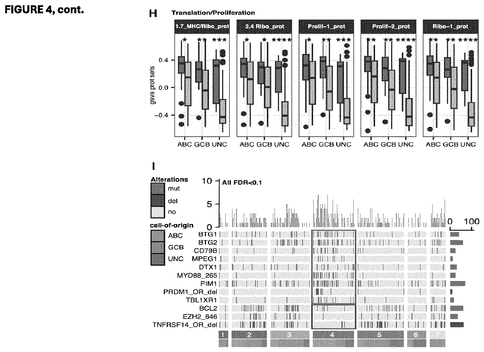

**Figure 4****: DLBCL proteogenotype 4 defined by immunosuppressive TME and deregulated cell-intrinsic pathways.** A) Differential testing for significant association of latent factors (LFs) with DLBCL proteogenotype 4 (PG4) status. The five LFs with logFC >0.3 and FDR < 0.01 are highlighted. B) Activity heatmap of the PG4 driving LFs. Rows represent LFs, columns the data layers. Each square color-codes the activeness of each LF (red: active; blue: inactive). The right annotation denotes the significant overlap with the prior *SignatureDB-based* information of each LF (significant overlap is highlighted by *, dark grey denotes significant overlap for both data layers, light grey only for the proteome). C/D) Bayesian-moderated gene-set enrichment analysis with *model-based gene set analysis* (*mgsa,* see methods) for the weights of LFs associated with PG4 that are active on the transcriptome (C) and the proteome (D). The gene ontology biological pathways and the *SignatureDB* gene set collections were used in C-D. E) Significantly differential average signature scores related to the tumor microenvironment (TME) based on the transcriptome (yellow) and significantly differential gsva scores derived from SignatureDB-corresponding phenotypic protein sets (blue) between PG4 and all other non-PG4 cases according to the cell-of-origin status. F) Similar to E, here for feature sets related to B-cell differentiation. G) Similar to E-F, for feature sets related to oncogenic signaling and transcription factor activity. H) Significantly differential gsva scores derived from SignatureDB-corresponding phenotypic protein sets (blue) between PG4 and all other non-PG4 cases for feature sets related to protein translation and proliferation. Statistical significance was determined in E-H by Wilcoxon-Mann-Whitney U test and multiple hypothesis correction using the Benjamini-Hochberg-Method. * P<0.05; ** P<0.01, *** p<0.001, **** p<0.0001. I) Oncoprint highlighting the significantly over- (red boxed) or underrepresented (blue boxed) mutations in PG4-DLBCL versus all others as determined by Fisher's test with a false-discovery rate <0.1 (Benjamini-Hochberg method). Top: Number of mutations per sample; right: Abundance of mutations across the discovery cohort (amp, amplification; del, deletion; mut, mutation).

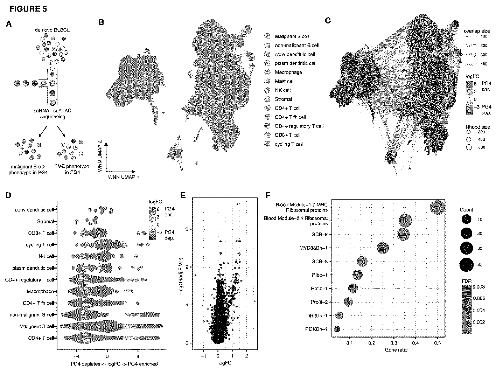

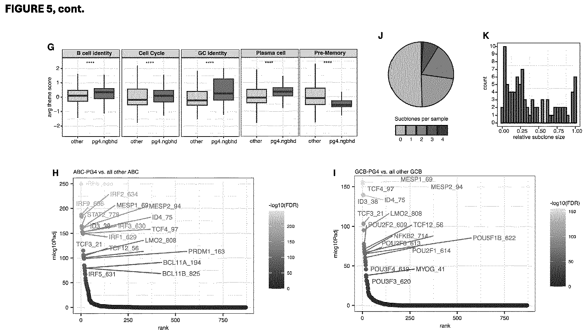

**Figure 5****: Single-cell gene expression and chromatin landscape in PG4-DLBCL.** A) Schematic visualization of single cellular characterization of malignant B-cell and TME-related T-cell phenotypes in PG4-DLBCL. B) UMAP visualization of *weighted-nearest-neighbor* integrated dimension of RNA expression and ATAC sequencing data of 197,860 single cells from 43 DLBCL patients of the validation cohort. Each dot denotes a single cell color-coded according to the assigned cell-types. C) Graph-based visualization of inferred single-cell neighborhoods. Each circle represents a neighborhood of single cells with circle sizes denoting the neighborhood sizes. Neighborhoods are color-coded for overrepresentation (red) or underrepresentation (blue) of cells found in PG4 compared to all other non-PG4 cases. Only significant over-/underrepresentations after multiple hypothesis correction by spatial FDR correction (FDR 0.1) are visualized. Graphical localization of each neighborhood is based on the UMAP representation in B. D) Assignment of inferred neighborhoods to their dominant cell type and visualized for under-/overrepresentation of cells found in PG4 versus non-PG4. E) Marker gene detection for PG4-overrepresented malignant B-cell neighborhoods in contrast to non-PG4 malignant B-cell neighborhoods. F) Hypergeometric testing for enrichment analysis of PG4 malignant B-cell neighborhood markers with the *SignatureDB* as feature set. G) Average gene expression theme scores for PG4-overrepresented malignant B-cell neighborhoods in contrast to non-PG4 malignant B-cell neighborhoods. Statistical significance was determined by Wilcoxon-Mann-Whitney U test and multiple hypothesis correction using the Benjamini-Hochberg-method. * p<0.05; ** p<0.01, *** p<0.001, **** p<0.0001. H) Transcription factor (TF) motif enrichment in PG4-ABC malignant B-cell neighborhoods in comparison to all other non-PG4-ABC malignant B-cell neighborhoods. TF-binding motifs are ranked by -log10(adjusted p-value). I) Similar to H, comparison of PG4-GCB malignant B-cell neighborhoods to all other non-PG4-GCB malignant B-cell neighborhoods. TF-binding motifs marked in red indicate shared representation in H-I. J) Number of PG4-predicted malignant B-cell subclones per sample. K) Relative size of PG4-predicted malignant B-cell subclones within one sample.

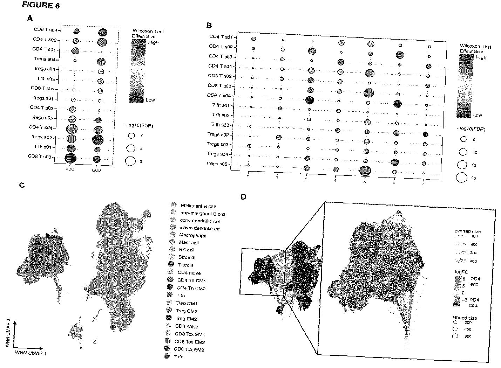

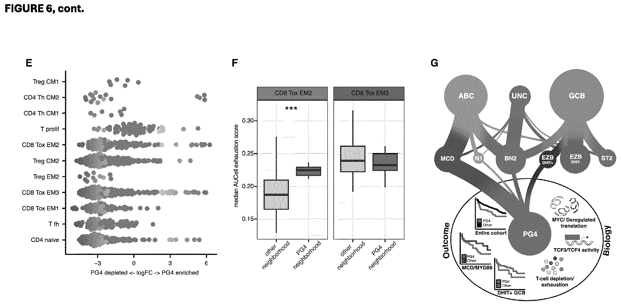

**Figure 6****: T-cell exhaustion and depletion in PG4-DLBCL.** A) Differential representation of T-cell states inferred by Lymphoma Ecotyper [3] between cell-of-origin subgroups. Color denotes the effect size, size highlights the level of significance (Wilcoxon test). B) Similar to A, tested for differences between all DLBCL proteogenotypes. C) UMAP visualization of *weighted-nearest-neighbor* integrated dimension of RNA expression and ATAC sequencing data (see Fig. 5b). Each dot denotes a single cell color-coded for the adapted cell type assignments. D) Graph-based visualization of inferred single-cell neighborhoods focusing on the T-cell compartment. Each circle represents a neighborhood of single cells with the size of the circle denoting the neighborhood size. Neighborhoods are color-coded for overrepresentation (red) or underrepresentation (blue) of cells identified in PG4. Only significant over-/underrepresentations after multiple hypothesis correction by spatial FDR correction (FDR 0.1) are visualized. E) Assignment of inferred neighborhoods to their dominant cell type and visualized for under-/overrepresentation in PG4. F) Mean AUCell score for exhaustion signature [2] in PG4 enriched vs. non-PG4 neighborhoods of CD8+ Tox EM2 and EM3 cell neighborhoods. Significance testing was performed by Wilcoxon test, *** p<0.001. G) Schematic visualization of clinical and biological PG4 characteristics.

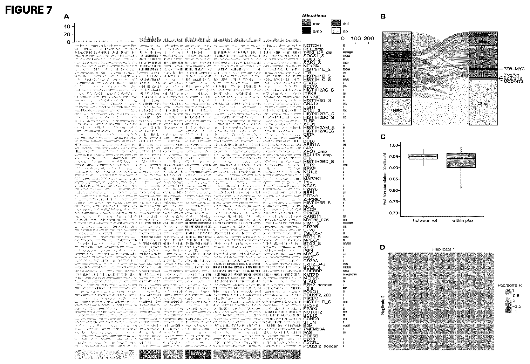

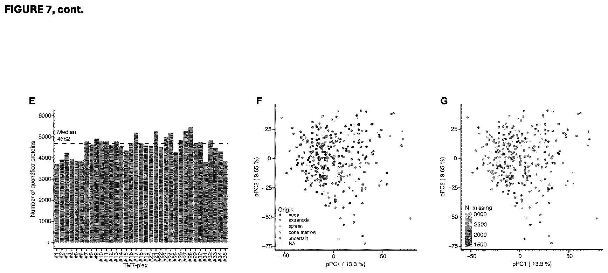

**Figure 7****:** A) Oncoprint of mutations per DLBCL case stratified by HMRN-based genetic subtypes. Top: Mutational frequency per sample, right: abundance of mutation across the study cohort (amp, amplification; del, deletion; mut, mutation). B) Comparison of HMRN and LymphGen-derived genetic subtypes of the discovery cohort. C) Pearson correlation of protein intensities across all reference channels of the entire cohort and within each analyzed TMT multiplex. D) Pearson correlation matrix for technical replicates of the mass spectrometry analysis. E) Number of identified and quantified proteins within each measured TMTmultiplex. Horizontal line indicates the median number of quantified proteins across all TMT multiplexes. F) Probabilistic principal component analysis (pPCA) of normalized protein intensity data with samples color-coded for the tissue origin. G) pPCA of normalized protein intensity data with samples color-coded for the number of missing proteins per individual sample.

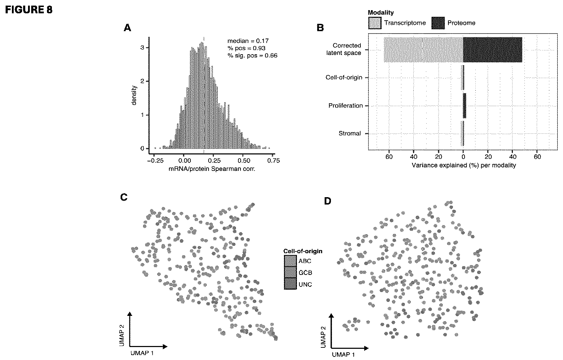

**Figure 8****:** A) Transcriptomic to proteomic expression correlation for all mRNA/protein pairs identified within the data sets and corrected for low abundant proteins (within 10^{th} percentile of global protein expression distribution). Positive correlations are highlighted in light blue, negative correlations in grey. The grey vertical line depicts the median Spearman's R for all RNA-to-protein pair correlations. B) Variance explained per modality of latent factors (LFs) associated with stromal, proliferation and cell-of-origin-features and the corrected latent space used for inference of the DLBCL proteogenotypes. C) UMAP visualization of the inferred multi-omic latent space related to cell-of-origin-associated LFs based on mRNA and proteomic expression data. Dots represent individual samples. D) UMAP visualization of inferred multi-omic latent space without LFs that are related to the cell-of-origin status.

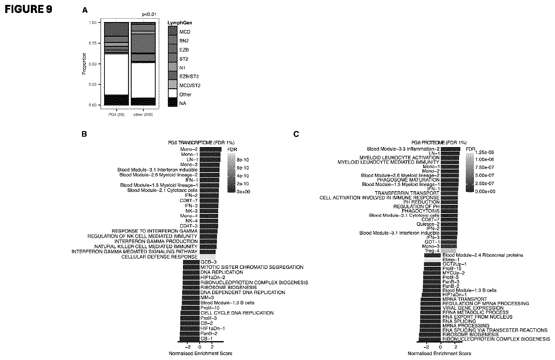

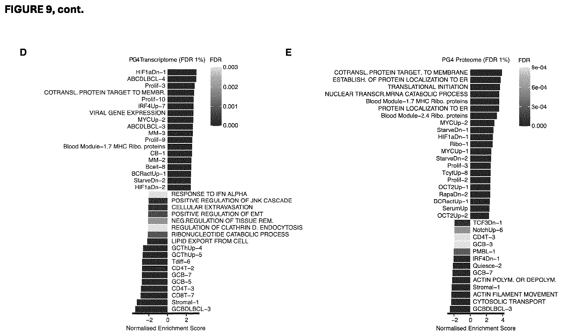

**Figure 9****:** (A) Distribution of LymphGen-based genetic subtypes in PG4 vs. all others. Statistical testing was performed for MCD in PG4 vs. others by Fisher's test. B/C) Gene-set enrichment analysis of differentially expressed genes (B) and proteins (C) for PG6 versus all others using the HALLMARK, gene-ontology biological pathways and *SignatureDB* gene-set collections. D/E) Gene-set enrichment analysis of differentially expressed genes (D) and proteins (E) for PG4 versus all others using the HALLMARK, gene-ontology biological pathways and *SignatureDB* gene-set collections.

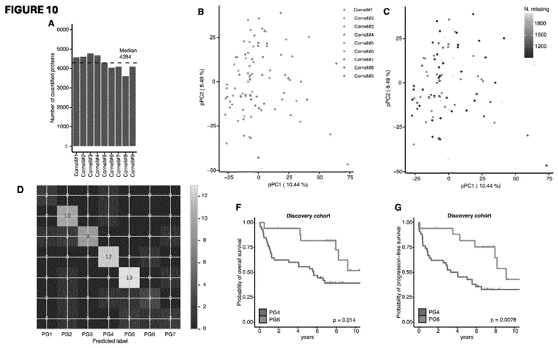

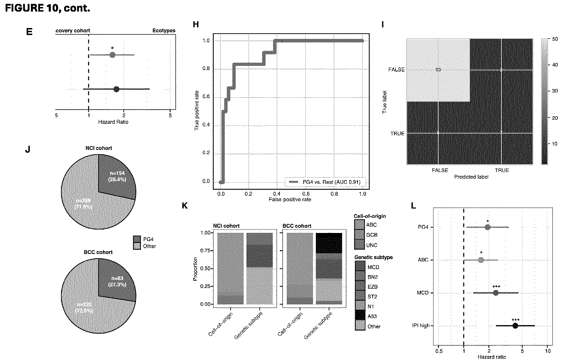

**Figure 10****:** A) Number of identified and quantified proteins within each measured TMT-plex of the validation cohort. Horizontal line indicates the median number of quantified proteins across all TMT-plexes. B) Probabilistic principal component analysis (pPCA) of normalized protein intensity data of the validation cohort. Individual samples are color-coded according to their TMT-plex. C) pPCA of normalized protein intensity data of the validation cohort with samples color-coded according to the number of missing proteins per individual sample. D) Confusion matrix for DLBCL PG predictions by classifier highlighted in Fig. 3 a-d. E) Multivariate cox-regression testing for predicted PG4 vs. all other DLBCL PG including the covariate Lymphome Ecotype (LE) 1 in all patients of the discovery cohort with DLBCL NOS and R-CHOP treatment. F) Overall survival (OS) estimate stratified by PG4 versus PG6 in all patients with DLBCL NOS and R-CHOP treatment. G) Progression-free survival (PFS) estimate stratified by PG4 versus PG6 in all patients with DLBCL NOS and R-CHOP treatment. H) Receiver operator characteristics (ROC) for prediction of PG4 from transcriptional data by the XGBOOST model trained on the discovery cohort. I) Confusion matrix for PG4 prediction from transcriptional data by the XGBOOST classifier. J) Proportion of PG4 predicted cases in NCI and BCC cohort. K) Distribution of cell-of-origin status and genetic subtypes (LymphGen) in PG4-predicted cases in the NCI and BCC cohort. L) Multivariate cox-regression testing for predicted PG4 vs. all other DLBCL PGs including the covariates ABC status, MCD status and IPI high status in the NCI cohort.

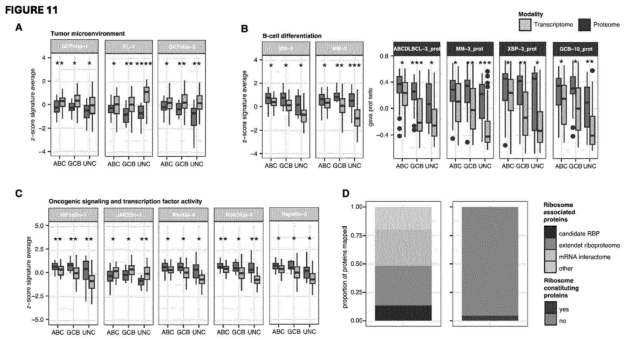

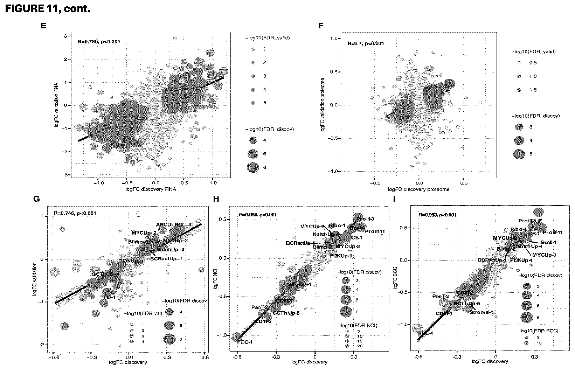

**Figure 11****:** A-C) Significantly differential average signature scores related to the TME (A), B-cell differentiation (B) and oncogenic signaling and transcription factor activity (C), based on the transciptome (yellow) and significantly differential gsva scores derived from SignatureDB-corresponding phenotypic protein sets (blue) between DLBCL proteogenotype 4 (PG4) and all other non-PG4 cases according to the cell-of-origin status. Statistical significance was determined in A-C by Wilcoxon-Mann-Whitney U test and multiple hypothesis correction using the Benjamini-Hochberg-Method. * p<0.05; ** P<0.01, *** p<0.001, **** p<0.0001. D) Proportion of proteins within the PG4 proteomic core signature assigned to the ribosome-associated riboproteome (left panel) or protein components of the ribosomes themselves (right panel) (RBP, RNA binding proteins). E) Correlation of the differential testing for the comparison of PG4 samples versus all others within the discovery and validation cohort transcriptomic data sets. Dot size denotes the significance in the discovery cohort data. Dot shade indicates significance in the validation cohort data. F) Similar to E, for the proteomic datasets of the discovery and validation cohorts. G) Correlation of the differential analysis of average signature scores for *SignatureDB* gene sets for the comparison of PG4 samples versus all others based on the discovery and validation cohort data sets. Dot size denotes significance in the discovery cohort. Dot shade indicates significance in the validation cohort data. H-I) Similar to G, now for the correlation of discovery cohort with NCI cohort (H) or BCC cohort (I).

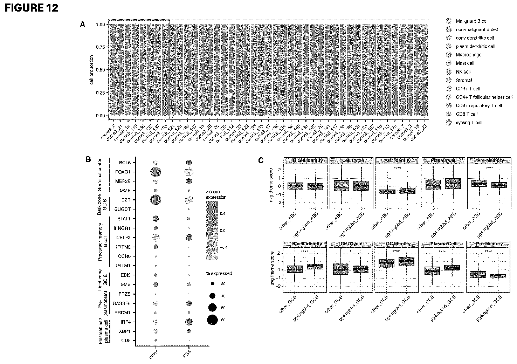

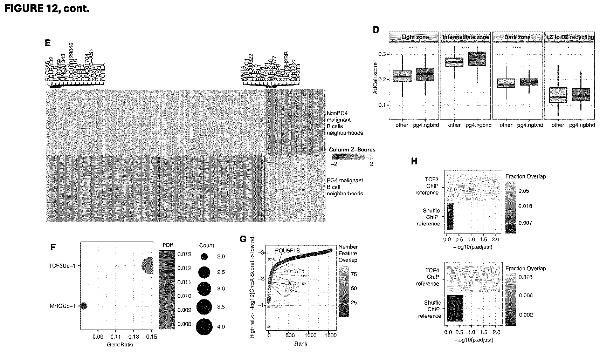

**Figure 12****:** A) Distribution of cell types across all samples. Green-boxed samples are predicted PG4-DLBCL cases. B) B-cell differentiation marker expression of PG4 malignant B-cells versus non-PG4 malignant B-cells. C) Average gene expression theme scores for PG4 overrepresented malignant B-cell neighborhoods in contrast to non-PG4 malignant B-cell neighborhoods. PG4-ABC (upper panel) and PG4-GCB (lower panel) malignant B-cell neighborhoods versus their respective non-PG4 counterpart malignant B-cell neighborhoods. Statistical significance was determined by Wilcoxon-Mann-Whitney U test and multiple hypothesis correction using the Benjamini-Hochberg-method. * p<0.05; ** P<0.01, *** p<0.001, **** p<0.0001. D) Median AUCell scores for germinal center light zone (LZ), intermediate zone (IZ) and dark-zone (DZ) feature sets as well as the LZ to DZ recycling phenotype in PG4 malignant B-cell neighborhoods versus all other non-PG4 malignant B cell neighborhoods. E) Gene score markers for PG4 malignant B-cell neighborhoods versus non-PG4 malignant B-cell neighborhoods. F) Hypergeometric testing for enrichment analysis of PG4 malignant B-cell neighborhood Gene score markers with the *SignatureDB* gene set collection as feature set database. G) Mean ChEA score for transcription factor relevance in PG4 core proteome feature set (Fig. 11d). Lower score indicates greater relevance, color indicates the number of proteins from the PG4 core proteome feature set present in the TF regulation feature set. Highlighted TFs were significantly enriched in the differential analysis highlighted in Fig. 5i-j. H) Enrichment PG4-predicted malignant B cell ATAC marker peaks in respective ChIP-sequencing reference peak collection for TCF3 and TCF4.

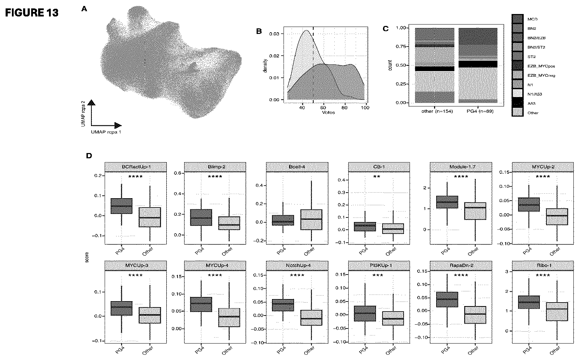

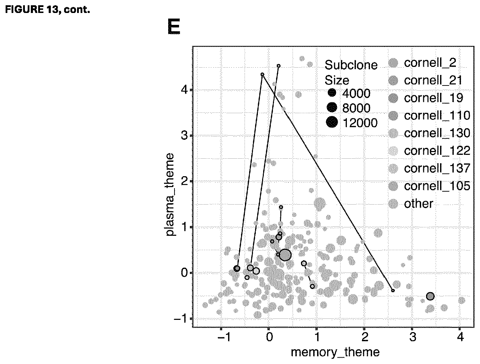

**Figure 13****:** A) UMAP visualization of integrated scRNA data from all malignant B cells. B) Distribution of PG-prediction votes (see methods) for either PG4 prediction (green) or other PG prediction by transcriptomic classifier on malignant B-cell subclones pseudobulk data. Horizontal dashed line indicates confidence cut-off for prediction. C) Distribution of genetic subtype background for PG4-predicted malignant B-cell subclones. D) Subclone-wise averaged gene-set scores per indicated gene-set from *SignatureDB.* Statistical testing was performed per Wilcoxon-test. * p<0.05; ** p<0.01, *** p<0.001, **** p<0.0001. E) Malignant B-cell subclones of PG4-predicted samples by multimodal classifier visualized for memory B cell and plasma cell gene expression theme. Size denotes the number of malignant B cells per subclone. Color indicates the samples. Subclones from the same sample are connected by black line.

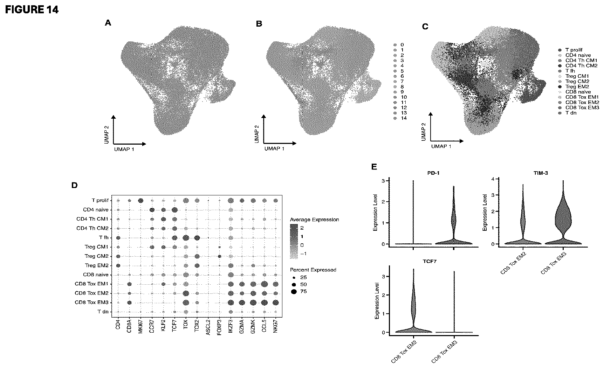

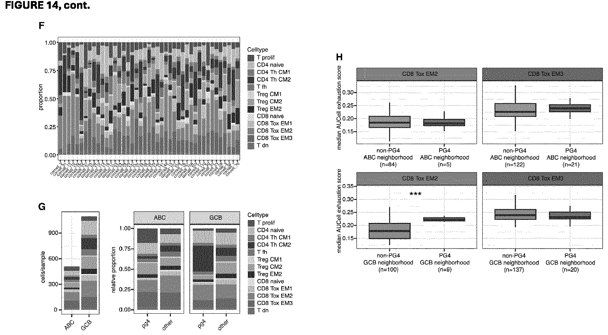

**Figure 14****:** A) UMAP representation of integrated T-cell RNA expression data (anchor-based integration) from 43 patients color-coded for patient ID. B) UMAP representation of integrated T-cell RNA expression data color-coded for identified graph-based clusters. C) UMAP representation of integrated - cell RNA expression color-coded for annotated T-cell clusters according to markers from [2]. D) Marker expression of annotated T-cell clusters. E) Expression levels of markers characterizing CD8+ Tox EM2 T-cells versus the exhausted EM3 population. F) Distribution of inferred T-cell clusters across all investigated patients. G) Absolute number/sample of T cells per T-cell compartment dichotomized for cell-of-origin status (left panel) and relative proportion of T-cell types compared between PG4 and non-PG4-predicted cases (multimodal classifier) in each cell-of-origin subtype (right panel). H) Median AUCell score for exhaustion signature ([2]) in PG4 vs. non-PG4 neighborhoods of CD8+ Tox EM2 and EM3 cell neighborhoods further stratified by cell of origin status (ABC/GCB). Significance testing was performed by Wilcoxon test, *** p<0.001.

In the following, reference is made to the examples, which are given to illustrate, not to limit the present invention.

### EXAMPLES

### Example 1:

Diffuse large B-cell lymphomas (DLBCL) are genetically and clinically heterogenous. Despite their genomic characterization, the pathophysiology of high-risk DLBCL remains incompletely understood. We report the first proteogenomic analysis of DLBCL tissues from 438 patients to elucidate disease pathophysiology and inform diagnostic and therapeutic approaches. By integration of transcriptome, proteome and mutation data, we identify seven DLBCL proteogenotypes (PG) reflecting specific pathophysiological features and spanning cell-of-origin (COO) boundaries. Remarkably, PG4 was discovered and validated as high-risk DLBCL independently of known risk factors such as COO, genetic subtypes and the International Prognostic Index. PG4 was enriched for subsets of activated B-cell-like DLBCL with MYD88/CD79b mutations and double-hit-signature-positive germinal-center-B-cell-like tumors. PG4 cases shared a dark-zone related B-cell phenotype and an enrichment of BTG1 and TBL1XR1 mutations. Single-cell sequencing of 43 cases revealed deregulated TCF3/4 transcriptional activity, enhanced BCR/MYD88 and PI3K signaling as well as a depleted and exhausted CD8 T-cell effector memory type 2 compartment as common driver mechanisms. Importantly, PG4 was detectable at the subclonal level providing evidence for intratumoral proteogenomic heterogeneity. Our study provides an integrated proteogenomic framework explaining so far unresolved disease heterogeneity and identifies molecular features defining high-risk DLBCL as a basis for innovative diagnostic and therapeutic approaches.

### Example 2: Results

### Multi-omics-based discovery of DLBCL proteogenotypes

For in-depth proteogenomic profiling, we characterized formalin-fixed paraffin-embedded (FFPE) tumor tissues from 332 DLBCL patients prior to R-CHOP-based first-line treatment (discovery cohort). Our real-world cohort included all COO subtypes and clinical risk categories (as determined by the international prognostic index (IPI), and showed clinical outcome comparable to previously published R-CHOP treated DLBCL cohorts (**Fig. 1a**). The COO subtypes were identified by the DLBCL automatic classifier. The individual genetic DLBCL subtypes were called by using the NCI LymphGen and UK HRMN classifiers, both showing a strong correlation, thus validating each other (**Fig. 1b****,** **Fig. 7a****-b**). Notably, due to absent copy number aberration data, the A53 genetic subtype could not be identified. In addition to genomics and gene expression, we extended our multi-platform approach by applying a tandem-mass-tag (TMT)-based quantitative mass spectrometry approach to elucidate the DLBCL proteome (**Fig. 1c****-d**). This approach yielded a high-quality proteomic data set with quantitative data for 6910 proteins, without relevant batch-effects related to the TMT-based multi-plex approach, tissue origin or missing-value patterns that can occur in large proteomic studies (**Fig. 1c****-e,** **Fig. 7c****-g**).

Previous studies reported a poor correlation of transcript and protein expression levels in different tumor types pointing towards a strong contribution of post-transcriptional mechanisms to the tumor pathophysiology. Therefore, we hypothesized that an integrated proteogenomic approach may have an added value over mere genomics to further resolve the biological and clinical heterogeneity of DLBCL. In line with previous studies, we also identified a low overall transcript-to-protein correlation for the detected transcript-protein pairs, also when removing low abundant proteins that may cause increased variability und thus lower correlation (**Fig. 1f****,** **Fig. 8a**). Notably, a subset of specific transcript-protein pairs defining cell-of-origin showed high correlation, indicating that these features are less dependent on post-transcriptional and translational processes (**Fig. 1g**).

For the integrated and in-depth proteogenomic characterization we next employed multi-view latent variable modeling (MuVI), a probabilistic approach guided by the extensive and informative knowledge on the feature sets driving inter-patient variability in lymphoma at the transcriptomic level (i.e. the lymphoma signature database [*SignatureDB*]) (**Fig. 1h**). In brief, MuVI integrates multimodal data by inferring interpretable latent factors, which are guided by lymphoma-related feature sets curated by the previously established SignatureDB. MuVI then infers which subset of these feature sets drive inter-patient variation in each modality and assigns a feature set-activity score for each patient. Further, it contextualizes modality-specific feature sets by adding and/or removing features to/from an active feature set that was initially defined by the *SignatureDB.* In our dataset, MUVI revealed 293 feature sets/latent factors driving molecular inter-patient heterogeneity. This included 11 feature sets related to cell-of-origin as a relevant source of variation, which recover the major gene expression subtypes, ABC and GCB-DLBCL (**Fig. 8b****-c**)**.**

In addition to COO, stromal and proliferation signals were identified to drive inter-patient variability, both known to correlate with survival of DLBCL patients upon R-CHOP-based treatment. To explore additional molecular structure beyond these three already established risk factors, that only explained a limited fraction (<10%) of the total variance in our multi-omics MuVI model (**Fig. 8b**), we focused on the 127 features sets/latent factors explaining relevant variance in the data and being not related to any of these themes mentioned above (**Fig. 8b****,d**)**.** We then performed graph-based consensus clustering on these remaining factors using graph-based community detection. This resulted in seven DLBCL proteogenotypes (PGs) that were robustly identified across all iterations during consensus clustering (**Fig. 1i****-j**).

As a result, disease-informed multi-modal integration of our proteogenomic data validated known molecular features of DLBCL (e.g. cell-of-origin); furthermore, it also revealed so far unexplored latent molecular representations that explain inter-patient variability and thus impact the proteogenomic classification of DLBCL. Consensus clustering based on proteogenomic features led to the identification of seven so far undescribed DLBCL PGs.

### Individual DLBCL proteogenotypes converge on specific molecular and cellular characteristics

Advanced profiling of DLBCL tumors has identified distinct molecular subtypes characterized by a great variety of molecular themes, some with prognostic and/or biological relevance [3]. To further explore the biological and clinical relevance of the identified PGs and investigating their relationship to previously described molecular patterns, we characterized the identified PGs for disease-related tumor cell-intrinsic and extrinsic features. PG1 (n=15, 4.5%) and PG7 (n=29, 8.7%) almost exclusively consisted of GCB cases and PG2 (n=56, 16.9%) was enriched for GCB cases with the EZB mutation profile. In contrast, PG6 (n=29, 8.7%) was enriched for unclassified (UNC) and PG4 (n=66, 19.9%) for ABC-DLBCL cases (the latter with relevant proportions of GCB and UNC cases present). The ABC-PG4 cases displayed a significant, but not exclusive, enrichment for the MCD/MYD88 genetic subtype (**Fig. 2a****-d**), while the GCB-PG4 cases were enriched for DHIT signature (DHITsig)-positive cases compared to all other (non-PG4) GCB cases (**Fig.2c****-d**, **Fig. 9a**). Notably, PG4 showed no significant overrepresentation for molecular high-grade features or FISH-confirmed double-hit lymphomas. Overall, no significant differences in the distribution of the *International Prognostic Index* (IPI) scores were observed among all the identified PGs (**Fig. 2a****-b**).

A recent characterization of DLBCL by the Lymphoma Ecotyper (LE) framework based on transcriptional data revealed cellular states reflecting the continuum of B-cell differentiation, some of which have prognostic relevance [3]. Moreover, the LE framework provides an estimate of the TME composition based on transcriptional expression data. Applying it to our transcriptomic gene expression data, we further elucidated the cellular states as well as TME features of the individual PGs (**Fig. 2a****-b**). Both, PG5 and PG6 were enriched for B-cell state 2, which is characterized by the expression of precursor memory B-cell markers including those from pre-memory B-cell subsets [3]. Although both were characterized by an inflammatory TME, their respective TME composition differed. PG6 showed an overrepresentation of LE4 characterized by a high abundance of CD4⁺, CD8⁺ and regulatory T-cells, as well as monocytes and macrophages (**Fig. 2a****-b, e-f,** [3]). The inflammatory TME of PG6 was independently validated when applying the CIBERSORT.x algorithm for digital cell-type deconvolution as well as gene-set variation analyses on the transcriptome and proteome (**Fig. 2e****-f,** **Fig. 9b****-c**). In contrast, PG5 was enriched for LE7 representing a TME dominated by dendritic and T-follicular helper cells **(****Fig. 2a****-b**, [3]). Moreover, PG5 also showed high mesenchymal, stromal and GC-like LME signature scores with a higher relative abundance of fibroblasts, endothelial cells and CD4+ T cells (**Fig. 2e****-f**).

PG3 and PG7 showed an overrepresentation of B-cell state 1, which represents a GC-related B-cell phenotype with favorable survival probability also highlighted by the high frequency of GCB cases in these PGs ([3], **Fig. 2a****-b**).

In contrast, PG4 was significantly enriched for LE1 representing B-cell state 5 with dark-zone GC B-cell- and plasmablast-derived gene and protein expression characteristics (**Fig. 2a****-b**, **Fig. 9d****-e**, [3]). PG4 also showed reduced CD4+ and CD8+ T cells and high scores for the depleted LME signature which suggests an overall less abundant immune microenvironment (**Fig. 2e****-f**).

Taken together, the identified PGs converge on specific biological patterns reflecting different lymphoma cell differentiation and mutation states, cell biological and oncogenic pathways as well as TME features. Hence, they provide a framework for exploration of disease pathophysiology beyond genomics.

### Independent validation of DLBCL proteogenotypes

To validate the biological and clinical characteristics of the DLBCL proteogenotypes, we characterized an independent cohort of 106 DLBCL patients treated at the Weill Cornell Medical College (USA) (further referenced as validation cohort) by RNA sequencing and mass spectrometry-based global proteome analysis and assured sufficient data quality **(****Fig. 10a****-c).** We then trained a gradient-boosted tree learning classifier on the transcriptomic and proteomic data-sets of our discovery cohort to predict PGs in the validation cohort in a multiclass-assignment approach (see methods). The resulting classifier model was first evaluated on a hold-out test set (from the discovery cohort) and displayed excellent performance metrics **(****Fig. 3a****,** **Fig. 10d****).** In the validation cohort data, 24.7% cases were assigned to PG4 with high confidence **(****Fig. 3b****).** To validate the assignments, the transcriptomic and proteomic datasets of the validation cohort were integrated using the previously mentioned MuVI approach. The resulting validation cohort was projected into the integrated latent space of the discovery cohort. Next, we quantified the similarity between the predicted PGs of the validation cohort and the corresponding PGs of the discovery cohort by computing the cohort-specific centroids of all PGs in the shared latent space **(****Fig. 3c****).** This revealed significantly shorter distances between the centroids of shared PG cases of the validation and discovery cohorts in comparison to non-shared PGs. This reflects that patients from the same PG were systematically mapped close in latent space, irrespective of whether they belonged to either the discovery or validation cohort, further validating the accurate prediction of PG4 by our multimodal classifier **(****Fig. 3d****).**

### PG4 predicts inferior patient outcome upon R-CHOP therapy

To investigate whether the individual PGs have prognostic relevance, we compared their clinical outcome. Remarkably, PG4 showed an inferior overall and progression-free survival (OS, PFS) compared to all other PGs of the discovery cohort **(****Fig. 3e****-h).** This was also evident after the correction of individual log-rank test models for multiple hypothesis testing. PG4 was furthermore identified as an independent risk factor for inferior OS in a multivariate analysis including ABC gene expression status and the IPI risk factors as covariates **(****Fig. 3i****).** Despite an overrepresentation of LE1 cases within PG4, the inferior OS of PG4 was also independent of the adverse survival associated with LE1 **(****Fig. 10e****).** In contrast, the immune-rich PG6 showed superior OS and PFS when compared to PG4 **(****Fig. 10f****-g).**

For further validation, we next explored other DLBCL cohorts with available transcriptome data for the presence of PG4 and its prognostic relevance. Therefore, we trained a gradient-boosted tree-learning model based on transcriptomic data from our DLBCL discovery cohort to predict PG4 using RNA expression data only. The resulting classifier showed excellent performance metrics when evaluated on the hold-out test set during the training process **(****Fig. 10h****-i).** We then applied this classifier to transcriptomic data of two independent DLBCL cohorts identifying predicted PG4 cases **(****Fig. 10j****,** BCC cohort). The resulting PGs showed similar distributions of the COO status and the LymphGen assignments as detected in our discovery cohort **(****Fig. 10k****).** Furthermore, predicted PG4 cases showed inferior outcome comparable to our discovery cohort, again independent of cell-of-origin (ABC), MCD mutation status or high IPI **(****Fig. 3j****-k,** **Fig. 10l****).**

### Molecular and TME characteristics of PG4-DLBCL

We next set out to perform a deep molecular characterization of PG4, with the identified pathway-based latent factors (LFs) as a starting point. We identified five LFs that explained variance between PG4 and all other PGs **(****Fig. 4a****-b),** which showed significant overlap with the prior feature set from *SignatureDB* after multimodal modeling. Importantly, only two of the PG4-driving LFs were active on the transcriptomic layer while all five LFs explained variance on the proteomic layer highlighting its added value for capturing relevant disease characteristics **(****Fig. 4b****).** Next, exploration of MuVI weights allowed to elucidate the transcriptome and proteome features of the PG4-defining LFs **(****Fig. 4b****-d).** The transcriptomic features were linked to a cellular differentiation state of naive B cells (Bcell-1) and CCR6+ memory B cell precursors (Bcell-4) **(****Fig. 4c****).** The five proteomic LFs revealed a more complex set of feature sets. All five LFs revealed a germinal center B cell motif with light and dark-zone features as well as an enrichment of terms related to ribosome biology and protein translation, both not being captured at the transcriptomic level **(****Fig. 4d****).** Notably, one of the LFs driven by proteomic information was associated with XBP1 target genes. During differentiation of B cells towards plasma cells, XBP1 coordinates the upregulation of ribosomal proteins and total protein synthesis. This is in line with the strong ribosome imprint and the B-cell differentiation signals driving the proteomic PG4-defining LFs **(****Fig. 4d****,** **Fig. 2a****).**

To further unravel the molecular and cellular characteristics of PG4, we next compared the averaged signature scores (average expression of each gene within on signature) derived from the transcriptomic expression data and the *SignatureDB* between PG4 and non-PG4 across all COO subgroups. Because transcriptomic information covered in the individual gene sets do not necessarily translate into similar proteomic feature sets (due to their overall low correlation), we translated the phenotypic transcriptomic information covered in the *SignatureDB* into informative protein sets to enable an informed exploration of the proteomic data (see methods). Here, several relevant motifs emerged with some being exclusive to one of the molecular data layers. First, a strong depletion of GC T follicular helper cell and follicular DC signatures in PG4 was evident in both data layers **(****Fig. 4e****,** **Fig. 11a****).** Second, PG4 showed an enrichment of signatures linked to CCR6+ memory B precursors (*Bcell-4*) and also (pre-) plasmablasts (*Blimp-2, Bcell-8*) with a DZ/centroblastic phenotype (*CB-1, GCB-10*) **(****Fig. 4f****,** **Fig.11b****).** Third, BCR (*BCRactUp-1*) and MYC (*MYCUp-2*/*3*) related signatures were observed in both ABC and GCB-PG4 cases suggesting that PG4 unites DLBCL from diverse COO backgrounds based on shared pathomechanistic features **(****Fig. 4g****,** **Fig. 11c****).** For instance, PI3K (*PI3KUp-1*) and mTOR (*RapaDn-2*) signaling were linked to PG4 across all COO subtypes **(****Fig. 4g****,** **Fig. 11c****).** In addition, enhanced OCT2 signaling activity (*OCT2Up-1*) was captured by proteomics only. This observation fits well with the enriched XBP1 signature in PG4, which is part of the OCT2-induced transcriptional program **(****Fig. 4g****).** Lastly, and as already revealed by exploring the PG4-defining LFs, PG4 was strongly enriched for ribosomal proteomic signatures across all COO subtypes **(****Fig. 4h****).** The respective proteins belonged to ribosome-binding-proteins, the mRNA interactome and the broader riboproteome as categorized by a previous mass spectrometry analysis of isolated ribosomal polysomes **(****Fig. 11d****).**

Next, we explored, whether specific mutations define PG4. ABC-PG4 cases showed an enrichment of mutations linked to the MCD and BN2 genetic subtypes **(****Fig. 4i****).** BTG1 mutations were enriched in both ABC- and GCB-PG4-DLBCL. Recently, BTG1 somatic mutations were shown to cause a supercompetitive malignant B-cell phenotype with induction of MYC-related biosynthetic programs that also include upregulation of relevant ribonucleotide and ribosome-associated proteins. This is in line with the PG4 phenotype described above. Moreover, mutations in were enriched which has been described as a key driver of lymphoma through induction of a pro-tumorigenic memory fate. In contrast, PG4 showed lower mutation frequencies for EZB-related mutations such as EZH2, BCL2 and TNFRSF14 **(****Fig. 4i****).**

Importantly, the identified molecular characteristics defining PG4 showed strong correlation when comparing PG4 cases in the discovery and multi-modal validation cohort, both on the transcriptomic and proteomic layers **(****Fig. 11e****-f).** When differentially comparing average signature scores generated from each cohort's transcriptomic data in PG4 versus non-PG4 cases, a high correlation was observed (R=0.746, p<0.001 for the Cornell validation cohort, R=0.956, p<0.001 for NCI cohort and R=0.963, p<0.001 for BCC cohort; **Fig. 11g****-i).** In all validation cohorts, PG4 was also characterized by enhanced MYC and BCR activity, increased expression of ribosome-related proteins, as well as a reduced abundance of GC T-follicular helper cells **(****Fig. 11g****-i).**

In summary, the identified proteogenotypes showed distinct clinical outcome with shortest PFS and OS observed for PG4-DLBCL. From a molecular perspective, PG4 was characterized by an immune cell-depleted TME, a MYC/BCR-driven lymphoma cell phenotype with deregulated expression of proteins regulating protein translation and specific genetic alterations including BTG1 and TBL1XR mutations.

### Transcriptional and TME attributes of PG4-DLBCL at the single-cell level

Single-cell RNA sequencing (scRNAseq) and exploration of chromatin accessibility by scATAC-seq have a proven potential to elucidate tumor pathophysiology because they allow to discriminate between cell-intrinsic and cell-extrinsic, TME-related processes. Motivated by our results from bulk proteogenomic profiling, we next conducted a single-cell sequencing approach to further explore the biology underlying PG4-DLBCL **(****Fig. 5a****).** We performed scRNAseq and scATACseq on tissue material from 43 DLBCL patients of the Cornell validation cohort. The COO subtype distribution within this set of samples was comparable to a general DLBCL population (ABC 34.9%, GCB 48.8%, UNC 16.3%). We applied our multiomic classifier-derived PG assignments and identified 8 of the 43 patients as PG4 (4 ABC, 3 GCB, 1 UNC). After stringent quality control of the sequencing data, 197,860 single cells with scRNAseq and scATACseq data available were explored further. Cell type annotation and identification of malignant B cells was performed. To integrate all available molecular information, we performed weighted-nearest-neighbor (WNN) analysis on the reduced dimensions from the scRNAseq and scATACseq data (see methods). This resulted in a WNN-graph of both molecular layers that was adjusted by cell-specific modality weights for each integrated modality (see methods). UMAP visualization of the inferred WNN-graph-based representation identified the TME compartment including CD4+ and CD8+ T cells as well as stromal and mast cell/macrophages in addition to the normal and malignant B cells **(****Fig. 5b****).** Overall, PG4 cases showed varying proportions of different cell types including malignant, stromal and immune cells **(****Fig. 12a****).** To study the cellular composition of PG4 tumors in more detail, we inferred cell neighborhoods on the basis of the integrated WNN-graph and tested for overrepresentation of cells from PG4 samples in each neighborhood (see methods, **Fig. 5c****).** This revealed a significant underrepresentation of cells stemming from PG4-DLBCL cases in the CD4+ and CD8+ T-cell neighborhoods, particularly in the CD4+ T follicular helper cell compartment, confirming our bulk proteogenomic data **(****Fig. 5c****-d).** We furthermore found cellular neighborhoods with an overrepresentation of PG4 malignant B cells **(****Fig. 5c****-d).** Enrichment analysis comparing the differentially expressed marker features between these PG4 and non-PG4 malignant B cell neighborhoods identified an enhanced mRNA expression of ribosome-associated gene sets in PG4 (*Module 1.7, Module 2.4, Ribo-1*)*,* as well as features related to germinal center and DHIT+/double-hit GCB DLBCL biology (*GCB-8, GCB-9*), MYD88/BCR signaling (*MYD88Dn-1*) and PI3K signaling (*PI3KDn-1*) **(****Fig. 5e****-f).** Only our single-cell analysis revealed that the molecular PG4 phenotype actually stems from the malignant B cell compartment. To gain further information regarding the cellular differentiation state of PG4 tumor cells, we investigated the expression of established B-cell lineage markers in the PG4 malignant B-cell neighborhoods compared to all other (non-PG4) neighborhoods [3]. PG4 malignant B cells showed in accordance with our bulk proteogenomics approach strong marker expression for germinal center, precursor memory B cell and pre-plasmablast differentiation **(****Fig. 12b****).** To further characterize the malignant PG4 B-cell neighborhoods, we applied established single-cell gene-expression theme scoring per cell. PG4 malignant B cells showed higher cell cycle activity as well as enhanced B cell receptor signaling and a cellular differentiation state as described above **(****Fig. 5g****).** Notably, these signals were concordantly observed for PG4 across all COO subtypes indicating a similar phenotype for PG4 malignant B-cells irrespective of their COO status **(****Fig. 12c****).** In addition, a LZ-to-DZ cycling and elevated LZ as well as DZ signals were observed in these PG4 malignant B cell neighborhoods **(****Fig. 5h****).**

Next, we investigated whether PG4 is associated with a specific chromatin accessibility pattern. Indeed, by gene expression estimation from the ATACseq-based chromatin accessibility data (see methods), we identified a set of chromatin characteristics specific for PG4 (compared to non-PG4 cases) that point towards hyperactivation of TCF3 (in GCB) and TCF4 (in ABC)-driven transcriptional programs. TCF3 is a transcription factor relevant for GC biology and often deregulated in Burkitt's lymphoma where its gain of function mutant variant (or inactivated ID3, a TCF3 inhibitor) significantly contribute to the pathophysiology of the disease **(****Fig. 12d****-e).** Similarly, TCF3 and ID3-related binding motifs were significantly enriched in PG4 malignant B cell neighborhoods **(****Fig. 5i****-j).** Strikingly, TCF3/ID3 and TCF4 were among the top predicted transcription factors to be responsible for the expression of the PG4-defining and ribo-enriched protein sets, thus linking TCF3/TCF4 activity to the PG4-defining proteome **(****Fig. 12f****).** In accordance with these results, we identified a significant enrichment of ATAC marker peaks in PG4 malignant B cells reflecting known TCF3 or TCF4 reference peaks defined by chromatin immunoprecipitation sequencing (ChIPseq) **(****Fig. 12g****).**

Having characterized lymphoma-cell intrinsic characteristics of PG4-DLBCL, we next explored whether PG4-like characteristics can be detected at the subclonal level. Therefore, we leveraged the single cell sequencing data of XX DLBCL cases with subclonal information. To identify PG4-like subclones, we pseudobulk-aggregated the single-cell transcriptomic data from all analyzed malignant B cells and subjected this to our transcriptomic classifier **(****Fig. 13a****).** We identified 89 of 243 malignant B-cell subclones to be PG4-like with high confidence **(****Fig. 13b****).** Importantly, around half of all samples studied contained at least one PG4-like predicted subclone which accounted in most cases for less than 25% of malignant B cells per sample **(****Fig.5j****-k).** These PG4 subclones had a similar genetic-subtype backgrounds as did PG4 cases in our bulk analysis **(****Fig.13c****).** Furthermore, the PG4-predicted subclones shared biological phenotypes regarding to BCR activity, MYC-related signals, PI3K-pathway activity and B-cell differentiation with the PG4 cases of our discovery cohort **(****Fig.13d****,** **e****).**

Taken together, the single cell sequencing analyses validated our bulk proteogenomics data and allowed to assign specific proteogenomic features to the malignant B-cell compartment. These include i) the differentiation status of PG4 malignant B cells as CCR6+ memory B precursors and also (pre-) plasmablasts with a DZ/centroblastic phenotype, ii) signaling features with enhanced MYD88/BCR and PI3K activity and iii) the increased expression of specific transcript/protein sets known as regulators of ribosome biology. The latter phenotype was captured only by proteomics, and not by transcriptomics, in the bulk analysis, suggesting that the transcriptional deregulation of this pathway as revealed by the single-cell analysis is further augmented on the proteomic level. Furthermore, deregulation of the TCF3 or TCF4 axis was identified as a key molecular feature defining the PG4 malignant B-cell compartment, thus likely contributing to the clinical high-risk phenotype of this particular DLBCL subset.

### PG4 is defined by an exhausted CD8 EM2 and EM3 T-cell compartment

Because our bulk proteogenomics data pointed towards an immunocompromised TME in PG4-DLBCL, we further explored our bulk and single cell-data regarding PG4-defining TME characteristics. The T-cell states as defined by the *Lymphoma Ecotyper* analysis showed the previously described differences in their abundance when comparing ABC versus GCB-DLBCL confirming once more the validity of our bulk proteogenomics data. Furthermore, PG4-DLBCL was almost depleted for T cells, in marked contrast to T cell-rich PG6 ([3], **Fig. 6a****-b).** To expand this analysis to single cell granularity, we integrated the scRNA expression data related to T cells data across all patients **(****Fig. 14a****).** ABC-DLBCL cases showed lower absolute numbers of T-cells per case compared to GCB-DLBCL **(****Fig. 14g****).** Graph-based clustering of the T-cell-related scRNA data resulted in 15 distinct T-cell clusters, which were annotated according to the recently established T-cell populations in B-NHL [2] **(****Fig. 14b-****d).** These correlated with the respective T-cell subset defining patterns described by [2] and allowed to discriminate between exhausted and non-exhausted T-cell phenotypes **(****Fig. 14d-****e).** Among the 43 DLBCL patients with single cell data available, a strong heterogeneity was observed regarding their intratumoral T-cell composition **(****Fig. 14f****).** We added this refined T-cell annotation to the entire single-cell data set of all analyzed cells, which enabled the interpretation of T-cell compartment differences among the individual PGs **(****Fig. 6c****-d).**

Here, we identified a strong depletion of most T-cell subsets in PG4 **(****Fig. 6d****-e).** However, even though overall T-cell frequencies were lower in PG4, certain T-cell subsets - especially the CD8+ Tox EM2 and EM3 compartment - showed strong overrepresentation in PG4 tumors. Interestingly, these overrepresented CD8+ T-cells showed a strong exhaustion phenotype supporting the notion that the inactivation of T-cell immunity is a key characteristic of PG4 tumors, irrespective of their COO status **(****Fig. 6f****,** **Fig. 14h****).**

Our study provides an integrated proteogenomic framework explaining so far unresolved disease heterogeneity and elucidates molecular features defining high-risk DLBCL. PG4 was identified as a high-risk DLBCL subset, spanning COO and genetic boundaries, but being defined by unifying cell-intrinsic and TME features. These include a deregulated TCF3/4 transcriptional program (most likely TCF4 in PG4-ABC and TCF3 in PG4-GCBs), enhanced BCR/MYD88 and PI3K signaling, increased expression of ribosome-associated effector proteins as well as an exhausted CD8 T-cell compartment. Importantly, and in line with the TCF3/4-related transcriptional phenotype, PG4 was defined by a dark-zone related B-cell phenotype in both GCB and ABC-DLBCL, the latter highly enriched for mutations in MYD88 and CD79B (MCD genetic subtype). This highlights that beyond the previous discovery of double-hit/dark-zone signature-positive high-risk GCB tumors, a major subset of ABC-MCD-DLBCL shows a similar B-cell phenotype and particularly poor outcome when compared to their non-PG4 counterparts. Overall, PG4-DLBCL is associated with poor outcome upon R-CHOP-based chemoimmunotherapy irrespective of COO, the MCD genetic profile or the IPI risk factors. Thus, early diagnostic identification of PG4-DLBCL and innovative therapeutic approaches based on the knowledge of disease pathomechanisms revealed in our study may improve patient outcome.

### Example 3: Methods

***Gene expression data.*** Genome-wide gene expression data in the discovery cohort was generated by the Illumina whole genome DASL (cDNA-mediated Annealing, Selection, Extension and Ligation). Preprocessing steps included variance stabilizing transformation, quantile normalization and summarization of probe levels expression to gene level. In the case of gene with more than one probe, summarization used the median value of more than two well-correlated probes (eliminating probes with mean Pearson correlation coefficient < 0.5 to other probes in the set). For RNA sequencing (RNAseq) of the validation cohort, paired-end 100 bp read sequencing was performed using the Illumina TruSeq V3 chemistry. Processing of the data included alignment to the human genome (NCBI build 38) using the STAR alignment pipeline. Normalization of raw counts was performed utilizing the variance stabilization transformation approach within the DESeq2 package.

***Panel-based mutation data.*** DNA was extracted from FFPE material with Qiagen QIAAmp DNA tissue kit. Indexed libraries were generated by using a modified version of the SureSelect XT Protocol (Agilent) and pooled (16plex). By biotinylated RNA baits (agilent Technologies), this approach covered 293 genes implicated in haematological malignancies using the SureDesing Interface. Captured libraries were quantified and sequenced on a Illumina HiSeq 2500 using 75 base paired-end sequencing. The average read depth was 500x reads per base. Short insert paired-end reads were aligned to the reference human genom (GRCh37) using the Burrows-Wheeler aligner. The variant calling was carried out by CaVEMan pipeline (Wellcome Trust Sanger Institute). Variant annotation was performed.

***Global proteome expression data.*** For global proteome expression profiling, proteins were extracted from FFPE tissue sections and subjected to LC/MS analysis. First, the sections were deparaffinized by incubating two times in 1 ml of each xylene and ethanol with shaking at 600 rpm. The samples were centrifuged for 5 min at 13,000 x g and the liquid was discarded, respectively. After deparaffinization, the samples were dried in a vacuum centrifuge and solubilized in tissue lysis buffer (4 % SDS, 0.2 % sodium-deoxycholate, 50 mM TCEP, 20 mM HEPES, pH 8.5) by shaking at 600 rpm and 90 °C for 60 min. The lysates were sonicated, centrifuged at 13,000 x g for 15 min and the supernatant was subjected to acetone precipitation overnight. After centrifugation at 13,000 x g for 20 min, the supernatant was removed and the samples were vacuum-dried. The protein pellet was solubilized in Urea buffer (8 M urea, 20 mM HEPES, pH 8.0, 1 mM sodium orthovanadate, 2.5 mM sodium pyrophosphate, 1 mM beta-glycerophosphate) and protein concentration was determined using Pierce A660 assay kit (Thermo Fisher Scientific). Subsequently, the protein samples were alkylated with iodoacetamide in a final concentration of 50 mM for 30 min at room temperature and protected from light, followed by enzymatic cleavage. For this, the samples were incubated with Lys-C (MS grade Lysyl Endopeptidase, Wako) in an enzyme-substrate ratio of 1:50 (w/w) for 2 h at 37 °C and, after dilution to 1 M Urea with 20 mM HEPES, pH 8.0, incubation was continued overnight with trypsin (MS grade, Promega) at 1:50 (w/w) enzyme-substrate ratio. For protein quantitation, the samples were labeled with tandem mass tags (TMT) according to the instructions of the manufacturer (Thermo Fisher Scientific) after determination of peptide concentration using Pierce fluorometric assay (Thermo Fisher Scientific). Briefly, 5 µg of peptides per sample were labeled with 50 µg of TMT 11-plex reagents for 1 h at room temperature and the reactions were quenched by incubation for 15 min with hydroxylamine in a final concentration of 0.2 %. Afterwards, each 11 individually labeled samples were mixed and the combined sample was dried by vacuum centrifugation. Thereby, each multiplex contained 5 µg of an internal reference sample that was prepared by mixing equal peptide amounts of each FFPE tissue sample and TMT-labeled accordingly (channel TMT10-126). For high pH reversed phase fractionation, the samples were solubilized in 20 mM ammonia solution (solvent A) and separated with a Waters XBridge C18 column (particle size 3.5 µm, dimension 1×150 mm) on an Agilent 1100 HPLC system using a stepped gradient to 90 % solvent B (20 mM ammonia solution, 80 % ACN). Over a duration of 70 min, 20 fractions were collected and consecutively combined to 10 samples following a scheme of 9 gaps (i.e. 1+11 and so forth). The peptide samples were vacuum-dried, solubilized in 0.1 % FA and subjected to LC/MS analysis in two replicate measurements on a nano-UHPLC system (Ultimate 3000 nRSLC, Thermo Fisher Scientific) interfaced with a quadrupole-Orbitrap hybrid mass spectrometer (Q Exactive HF, Thermo Fisher Scientific) through a nano-ESI ion source (Nanospray Flex, Thermo Fisher Scientific). After purification and concentration on a C18 pre-column (5 cm, packed with ReproSil-Pur 120 C18-AQ, 5 µm particle size, Dr. Maisch GmbH), the samples were separated on a C18 analytical column (32 cm, packed with ReproSil-Pur 120 C18-AQ, 1.9 µm particle size, Dr. Maisch GmbH) using a linear gradient of 2 to 40% solvent B (80 % ACN, 0.1 % FA) against solvent A (0.1 % FA) at a flow rate of 300 nl/min over 118 min. Eluting peptides were ionized and analyzed by tandem MS (MS/MS) using a data-dependent acquisition scheme. Survey scans were recorded using the Orbitrap mass analyzer in the range of m/z 350-1600 with a resolution setting of 120,000 FWHM at m/z 200 and considering charge states 2-6. The 20 most abundant precursor (peptide) ions were quadrupole-selected in an isolation window of m/z 1.4 for higher-energy collision-induced dissociation (HCD) with a normalized collision energy of 32% and nitrogen as collision gas. The fragment ions were analyzed using the Orbitrap in the range of *m*/*z* 200-2000 with a resolution setting of 60,000 FWHM at *m*/*z* 200. AGC target values and maximum injection times for MS and MS/MS were set to 1×10^6 in 40 ms and 1×10^5 in 64 ms, respectively. Precursor ions that had been selected for HCD were excluded from repeated isolation for 20 s.

For protein identification and quantitation, the MS raw data was processed with version 2.0.3.0> of the MaxQuant software (PMID 27809316) and the LC/MS runs of the individual 10 fractions per sample were merged, but keeping the replicate injections separate. Utilizing the integrated Andromeda peptide search engine, mass spectra were matched against the Uniprot human protein database (Swiss-Prot/TrEMBL, release 04-2021, 79,057 entries) and a collection of frequently observed laboratory contaminants (246 entries). The mass tolerances were set to 4.5 ppm and 20 ppm for precursor and fragment ions, respectively. Oxidation of methionine and acetylation of the protein N-terminus were considered as variable modifications and carbamidomethylation of cysteine was defined as fixed modification. The minimal peptide length was set to seven amino acids and to up to two missed tryptic cleavage sites were allowed. Both peptide and protein FDR were set to 0.01 using a decoy approach by searching the reversed database. For TMT-based protein quantitation, the extraction of reporter intensities on the MS2 level was enabled and correction factors for isotopic impurities of TMT labeling reagents were considered. The initial MaxQuant output data was filtered by removing potential contaminants, hits to the decoy database and proteins identified solely based on modified peptides. The TMT quantitation data was adjusted for equal peptide loading within multiplexes by standardization on the median summed-up reporter intensities of each TMT channel (intra-plex) and scaled between individual samples according to the internal reference (inter-plex).

### QUANTIFICATION AND STATISTICAL ANALYSIS

***Transcriptome to proteome correlation analysis.*** The normalized transcriptomic and proteomic expression dataset were queried for matching protein/gene names. To minimize the impact of proteomic missing value distribution, the proteomic expression dataset was filtered for 50% completeness. Both datasets were scaled to unit variance in a feature-wise manner, and the pairwise complete observation Spearman correlation coefficient was computed for each mRNA/protein pair (matched by HUGO symbols) across all patients. To quantify the extend to which mRNA/protein pairs were consistently differentially expressed between cell-of-origin status, an additional correlation analysis was performed. First, for both data modalities features that were differentially expressed between cell-of-origin status were identified based on a linear model (implemented using the R package limma). For the proteomic model, the TMT-plex information was added as an additional batch covariate to the model's design matrix. To compare general trends in terms of differential expression patterns, the Spearman rank correlation coefficient was computed for the log fold changes (logFC) of all matched mRNA and protein features (*all*)*.* Further, only significantly differentially expressed features (logFC >0.2/<-0.2 and FDR 0.02) were selected and correlated (Spearman correlation, ABC vs. *GCB sig*)*.*

***Data imputation.*** Mostly, unimputed data was used for subsequent analysis to avoid any bias. If analyses required a complete dataset without missing values, the DreamAI algorithm (Ma W, BioRxiv, 2021) representing an ensemble approach including k-nearest neighbor, random forrest, matrix factorization and ridge regression was applied. Input data was filtered to 50% missing value rate and the default parameters were used.

***Multi-omics data integration by MuVI.*** We applied the recently proposed MuVI algorithm, based on a group factor analysis model with informed structured sparsity, to integrate multimodal data and infer interpretable latent factors. We included the top 6000 most variable features in each modality and performed a view-wise data normalization to unit variance and a feature-wise centering of the data. The latent factors were explicitly informed by lymphoma-related pathways curated by the previously established Signature DB to ensure the inherent interpretability of the results. We trained MuVI with 281 latent factors, comprising 279 informed factors, and 2 uninformed factors, and used the default training parameters. In brief, we set alpha to 0.01, which controls the penalty to the prior information; we iterated over 10000 epochs with a decaying learning rate starting at 0.05 and trained until the optimization objective no longer improved in consecutive iterations.

***Consensus-based inference of proteogenotypes.*** The integrated multi-omics latent space was corrected for known confounders by removing latent factors (LF) that associate with stromal and proliferation information as well as cell-of-origin-associated LFs. This corrected latent space was then used as input to a consensus clustering approach via iterative community detection, adapting a consensus clustering strategy. First, the Leiden algorithm was run for 1000 iterations with random seeds on the corrected latent space. The results were then combined into a cluster assignment matrix where each column denotes a sample and each row the community assignment by the respective iteration. This matrix was then used for repetitive clustering with a random subsets of rows and columns (drawn randomly without replacement from the full assignment matrix) using the hamming distance and hierarchical clustering with complete linkage each time. A consensus partitioning analysis was then applied to infer the optimal number of subgroups and assess the robustness of the respective partitions. A consensus matrix was computed to quantify how consistently two samples are attributed to the same cluster. Each entry in the consensus matrix corresponds to the probability of a pair of samples belonging to the same cluster for each iteration. This was implemented using the R package cola, via the *hamming.distance()* function and hierarchical clustering via *hclust()* as part of the function *consensus_partition().*

***Probabilistic principal component analysis.*** To delineate principal components of variation in the global proteome expression data (with missing values), the normalized data was scaled to unit variance and zero mean in a feature-wise manner and a probabilistic PCA was performed using the "ppca" method within the *pcaMethods* R package.

***Differential expression analysis.*** To identify differentially expressed features - either between the proteogenotypes or for one PG versus all others - we leveraged an empirical Bayesian approach which regularizes the feature-wise residual variances towards a common value. This analysis is implemented within the R package *limma's* eBayes() method.

***Gene set enrichment analysis.*** To investigate enriched gene/protein sets, the differential expression results were ranked by the empirical Bayes moderated t-statistic which were input to the *fgseaMultilevel()* function of the R package *fgsea* with the indicated gene set collections. The resulting enrichment results were filtered for the indicated false-discovery-rate (FDR) cut-off, ranked by the normalized enrichment score and most-relevant top/bottom-terms were visualized.

***Gene-set analysis of MuVI factor weights.*** To characterize the pathways associated with a MuVI factor of interest, we ranked features (genes/proteins) by factor weights and selected the top and bottom 500 features for pathway enrichment analysis. To account for the redundancy of shared genes across gene sets, we leveraged a Bayesian model-based approach where the model infers probabilities of each gene set to be active within the given observations (genes). This analysis is implemented in the R package *mgsa.* Gene Ontology biological pathways (BP) and SignatureDB were used as gene set collections.

***Gene set variation analysis.*** To generate a feature-set-by-patient representation, we utilized a non-parametric, unsupervised gene set enrichment variation estimation implemented in the R package *gsva.* Here, either the normalized whole-genome panel-based gene expression data or the imputed global proteome expression data was used as input together with the indicated gene-sets.

***Inference of phenotypic protein sets.*** To derive protein sets that associate with a certain DLBCL-related phenotypic information, a protein set database (ProteinPhenotypeDB) was generated. In detail, the transcriptomic dataset was used to generate average signature scores for each sample and each gene set within the SignatureDB. Next, samples were ranked according to their average signature score for this gene set from high to low. The 10% of samples with the highest signature scores (Top 10%) and the 10% of samples with the lowest signature scores (Bottom 10%) for each signature were identified. Then, a differential protein expression analysis between the top 10% and bottom 10% samples per phenotypic signature was performed. Proteins found to be significantly upregulated (logFC > 0, FDR < 0.05) were then grouped into the phenotypic protein set. This was repeated for each of the gene sets within the SignatureDB, resulting in a corresponding phenotypic protein set data base (ProteinPhenotypeDB). The ProteinPhenotypeDB collection was then used as input for GSVA on the imputed protein data to assign phenotypic protein GSVA scores to each of the measured samples, which further were compared across the proteogenotypes and cell-of-origin status.

***DHITsignature prediction.*** To predict the DHITsignature positivity status of GCB cases in the study cohort, the self-training implementation of probability ratio-based classification prediction score (PRPS-ST) was used. In detail, the genes with weights from the initial DHITsignature classification present in the transcriptomic expression data of the study cohort were used. PRPS-ST was run with default parameters (ratio search range from 0.05-0.95, probability cut-off 0.9).

***Signature average scores.*** Signature averages were computed as described previously. In brief, the mean expression for all genes within one gene set was calculated for each gene sets and these values were standardized to zero mean and standard deviation of 1.

***Digital cell deconvolution by CIBERSORT.x.*** The genome-wide panel-based gene expression data was input to the CIBERSORT.x algorithm. To minimize platform-differences-dependent batch effects between the signature matrix (sc-RNA-seq) and mixture samples (DASL RNA array), bulk-mode batch correction was applied to remove technical differences. Since the mixture file does not stem from RNA-seq data, quantile normalization was not disabled. To acquire a deconvolution p-value, 500 permutations were performed. LM22 signature matrix (22 human immune cell subsets) and TR4 signature matrix (epithelial, endothelial, immune and fibroblast populations) were used and immune cell populations from LM22 signature were normalized to the immune fraction in TR4. All cell fractions are normalized in the end to 100%.

***Machine-learning classifier training.*** The multi-class classifier comprises a two-step approach with a feature elimination and a subsequent ensemble classifier. First, we perform an 80/20 balanced split into training and test sets. The training set then undergoes a recursive feature elimination with a five-fold nested cross-validation based on the feature importance scores provided by XGBoost classifiers. Specifically, we further split the training data into one outer test split and five training splits for each outer fold, which serve as the input for the inner cross-validation part. Each outer fold generates a set of 200 predictive features, which are then aggregated as the union of the features selected in at least two of the five folds. We perform the same procedure independently 10 times at random and take the union of the features selected from each trial, resulting in 3483 transcriptomic and 1990 proteomic features. We evaluate the procedure on the global test set. As a second step, we employ an ensemble classifier consisting of 100 XGBoost base models, each trained on a subset of 200 random features from the training data. Each XGBoost base model assigns a probability distribution over the seven proteogenomic groups during the predictive task. Based on a majority voting approach we aggregate the results over all base models and assign a final prediction to each sample in a given dataset. The training and the test data are z-scored to ensure the transferability of the predictive power among different data distributions.

### Processing and reciprocal PCA integration of single-cell RNA expression data.

Each sample's single-cell RNA expression data was processed individually controlling for the number of unique genes detected per cell (100-10000), the number of reads/counts (100-10000) and the percentage of mitochondrial counts (<30%) per sample. Further, log-transformation was performed to normalize the data, top 2000 most variable genes were identified and a PCA-dimension reduction per patient applied. Then, each dataset was projected into the others' PCA space and anchors with the same mutual neighborhood requirement were constrained (reciprocal PCA). The similar approach was performed for the integration of all analyzed T cells **(****Fig.6****).**

***Processing and integration of single-cell ATAC sequencing data.*** To process the single cell ATAC sequencing (scATAC-seq) data, the ArchR framework was used. Here, each sample underwent a strict QC controlling the number of unique nuclear fragments (>1000), the signal-to-background ratio defined by the TSS enrichment score (>4) and the relevant fragment size distribution. Additionally, the scATAC-seq data was curated for doublet measurements by ArchR's inherent doublet identification and subsequent removal method. Given the sparse data property of scATAC-seq data, latent semantic indexing (LSI) was used to reduce the dimensionality of the sparse insertion counts matrix. The Harmony algorithm was used to perform batch correction on the inferred LSI embedding.

***Multimodal integration of single-cell RNA expression and ATAC sequencing data.*** Weighted nearest neighbor (WNN) analysis was used to integrate the PCA and batch-corrected LSI representations from both single-cell modalities.

***Single-cell differential abundancy analysis.*** To compare cell type abundancies across PG4 and non-PG4 samples, we utilized the miloR framework. 5% of all cells (*prop*=*0.05* in *makeNhoods()*) were sampled using a KNN sampling algorithm based on the WNN graph to serve as set of index cells. Then, the number of cells from each experimental condition (PG4 vs. nonPG4) was counted for each neighborhood. To test for the differential abundance, a negative binomial generalized linear model was fit for the counts of each neighborhood, accounting for the different numbers of cells across samples. To control for multiple testing, a spatial FDR correction was applied. Here, the p values were weighted by the reciprocal of the distance to the kth nearest neighbor. For visualization purposes, neighborhoods were assigned to a respective cell-type if more than 80% of cells in this neighborhood came from a shared cell-type. To additionally infer markers for those neighborhoods that represent significantly enriched PG4-stemming malignant B cells, gene expression across each neighborhood was mean averaged post-hoc neighborhood markers were identified via *findNhoodGroupMarkers().* These were then input to a hypergeometric overrepresentation analysis performed with the *SignatureDB* gene set collection.

***Single-cell gene expression theme analysis.*** Gene weights per gene expression theme were generated by selecting all genes within a theme and extracting the factor loadings from the first principal component derived from a PCA on the single-cell RNA expression dataset limited to the genes within the respective theme. Applying *Seurat's AddModuleScore()* function, feature set scores per cell were generated by averaging the expression levels of genes within a theme and then subtracting the aggregated expression of simulated matched control themes. This was performed separately for genes with a positive or negative weight in the first PC of the corresponding theme based PCA for all positively and negatively weighted genes. Then, the results were multiplied by the sum of associated gene weights per theme. Finally, the negative weighted scores were subtracted from the positive weighted scores and z-score scaling per theme across cells was used to visualize the respective theme scores. Here, the z-scores for each neighborhood were averaged and the comparison was performed via Wilcoxon-test between PG4-enriched neighborhoods and non-PG4-neighborhoods among malignant B cells. To investigate the effects in each cell-of-origin subgroup, the z-scores for each cell-of-origin subgroup in each neighborhood were averaged and compared as before but now within each cell-of-origin subgroup.

***T-cell exhaustion scores.*** AUCell was used to infer the T-cell exhaustion scores for the respective gene signature from [2] and average AUCell scores were computed for each T-cell neighborhood [2].

***Gene score prediction, Peak calling, transcription factor motif enrichment and ChIPseq peak overlap analysis.*** Gene score predictions were inferred for the malignant B cell populations (via *addGeneScoreMat()*) with standard settings and gene score markers for the comparison of PG4-neighborhood stemming versus non-PG4-neighborhood stemming malignant B cells were detected (log fold change > 1 and FDR ≤ 0.1). Peak calling was performed with default settings utilizing the MACS2 pipeline. Transcription factor motif enrichment in PG4-neighborhood malignant B cell marker peaks was performed by hypergeometric enrichment analysis within each cell-of-origin subtype (ABC and GCB). The identified motifs were visualized after ranking by FDR. To identify a relevant overlap of identified marker peaks with published ChIP-sequencing datasets of TCF3 and TCF4, the genomic coordinates of the reference ChIP-peaks were flipped over to hg38. *enrichPeakOverlap()* of the ChIPseeker package (v.1.38.0) was used to generate a background null distribution by shuffling the coordinates of the reference peaks and comparing this to the query peak set (ATAC marker peak coordinates for PG4 malignant B cells) with the target peak set (either TCF3 or TCF4 ChIP peak coordinates).

***Survival analysis.*** Kaplan-Meier estimates were computed for the indicated contrasts. Differences in survival estimates were tested by the log-rank test. The univariate log-rank tests run for each proteogenotype against all others were corrected for multiple hypothesis testing via the Benjamini-Hochberg method. To test for the independence of the inferior survival phenotype in PG4 from cell-of-origin or IPI-status, a multivariate cox-regression model was fit with PG4 vs. rest and ABC, GCB and IPI status as covariates. The optimal penalization term which yielded the highest C-index as the goodness-of-fit was inferred with 8-fold cross validation.

***General statistical methods.*** R version 4.2.0 (local machine) and 4.2.2 (computation cluster) (The R Foundation for Statistical Computing) was used for statistical analysis. If not otherwise outlined, continuous variables were compared with the Mann-Whitney-U test for two independent groups and Kruskal-Wallis test for three or more independent groups, categorical variables with the Fisher's exact test and the chi-square test as indicated.

### REFERENCES

[1] Alduaij W, Collinge B, Ben-Neriah S, Jiang A, Hilton LK, Boyle M, Meissner B, Chong L, Miyata-Takata T, Slack GW, Farinha P, Craig JW, Lytle A, Savage KJ, Villa D, Gerrie AS, Freeman CL, Gascoyne RD, Connors JM, Morin RD, Sehn LH, Mungall AJ, Steidl C, Scott DW. Molecular determinants of clinical outcomes in a real-world diffuse large B-cell lymphoma population. Blood. 2023 May 18;141(20):2493-2507. doi: 10.1182/blood.2022018248. PMID: 36302166.
[2] Roider, T., Baertsch, M.A., Fitzgerald, D., Vohringer, H., Brinkmann, B.J., Czernilofsky, F., Knoll, M., Llao-Cid, L., Mathioudaki, A., Fassbender, B., Herbon, M., Lautwein, T., Bruch, P.M., Liebers, N., Schurch, C.M., Passerini, V., Seifert, M., Brobeil, A., Mechtersheimer, G., Muller-Tidow, C., Weigert, O., Seiffert, M., Nolan, G.P., Huber, W., and Dietrich, S., Multimodal and spatially resolved profiling identifies distinct patterns of T cell infiltration in nodal B cell lymphoma entities. Nat Cell Biol, 2024. 26(3): p. 478-489
[3] Steen, C.B., Luca, B.A., Esfahani, M.S., Azizi, A., Sworder, B.J., Nabet, B.Y., Kurtz, D.M., Liu, C.L., Khameneh, F., Advani, R.H., Natkunam, Y., Myklebust, J.H., Diehn, M., Gentles, A.J., Newman, A.M., and Alizadeh, A.A., The landscape of tumor cell states and ecosystems in diffuse large B cell lymphoma. Cancer Cell, 2021. 39(10): p. 1422-1437 e10

The features of the present invention disclosed in the specification, the claims, and/or in the accompanying figures may, both separately and in any combination thereof, be material for realizing the invention in various forms thereof.

## Claims

1. A method for the diagnosis, prognosis, and/or stratification of a diffuse large B-cell lymphoma (DLBCL) of a patient, comprising the steps of
a) providing a sample of said patient,
b) determining whether said sample comprises at least two, preferably at least three, more preferably at least four, even more preferably all of features i)-v):
i) a genotype comprising
an activated B-cell genotype with a MYD88 L265P mutation and a CD79B mutation, or
a double-hit signature positive germinal center B-cell genotype;
wherein, preferably, said genotype further comprises a BTG1 mutation and/or a TBL1XR1 mutation;
ii) a dark-zone phenotype;
iii) an enhanced signaling of TCF3, TCF4, MYC, BCR, MYD88, and/or PI3K compared to a control; wherein, preferably, the control is a reference sample, a collection of reference samples, a reference value, or a collection of reference values; wherein, more preferably, the control is a reference sample, a collection of reference samples, a reference value, or a collection of reference values of a healthy individual, a healthy cohort, or a cohort of DLBCL patients;
iv) an enhanced expression of one or more, preferably a plurality of proteins selected from ribosomal proteins and ribosome-associated proteins, preferably ribosomal proteins, compared to the control;
v) a tumor microenvironment **characterized by** depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk diffuse large B-cell lymphoma (DLBCL) in said patient.

2. The method according to claim 1, wherein said determining in step b) comprises determining whether said sample comprises an enhanced signaling of TCF3, TCF4, MYC, BCR, MYD88, and/or PI3K compared to the control;
wherein, preferably, said determining comprises determining whether said sample comprises an enhanced signaling of at least two, preferably at least three, more preferably at least four, even more preferably all of TCF3, TCF4, MYC, BCR, MYD88, and PI3K compared to the control.

3. The method according to claim 1 or 2, wherein step b) comprises determining whether said sample comprises at least two, preferably at least three, more preferably at least four, even more preferably all of features i)-v):
i) a genotype comprising
an activated B-cell genotype with a MYD88 L265P mutation and a CD79B mutation;
wherein, preferably, said genotype further comprises a BTG1 mutation and/or a TBL1XR1 mutation;
ii) a dark-zone phenotype;
iii) an enhanced signaling of TCF4, MYC, BCR, MYD88, and/or PI3K compared to the control, preferably of TCF4, MYC, BCR, MYD88, and PI3K compared to the control;
iv) an enhanced expression of one or more, preferably a plurality of proteins selected from ribosomal proteins and ribosome-associated proteins, preferably ribosomal proteins, compared to the control;
v) a tumor microenvironment **characterized by** depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk DLBCL in said patient.

4. The method according to claim 1 or 2, wherein step b) comprises determining whether said sample comprises at least two, preferably at least three, more preferably at least four, even more preferably all of features i)-v):
i) a genotype comprising
a double-hit signature positive germinal center B-cell genotype;
wherein, preferably, said genotype further comprises a BTG1 mutation and/or a TBL1XR1 mutation;
ii) a dark-zone phenotype;
iii) an enhanced signaling of TCF3, MYC, BCR, and/or PI3K compared to the control, preferably of TCF3, MYC, BCR, and PI3K compared to the control;
iv) an enhanced expression of one or more, preferably a plurality of proteins selected from ribosomal proteins and ribosome-associated proteins, preferably ribosomal proteins, compared to the control;
v) a tumor microenvironment **characterized by** depleted T cells compared to the control, preferably by depleted CD8+ effector memory T-cells compared to the control;
wherein the presence of at least two, preferably at least three, more preferably at least four, even more preferably all of features i)-v) in said sample of said patient is indicative for the presence of a high-risk DLBCL in said patient.

5. The method according to any one of the foregoing claims, wherein said determining in step b) comprises determining whether said sample comprises an enhanced expression of one or more, preferably a plurality of proteins selected from ribosomal proteins and ribosome-associated proteins, preferably ribosomal proteins, compared to the control.

6. The method according to any one of the foregoing claims, wherein said determining in step b) comprises determining whether said sample comprises a tumor microenvironment **characterized by** depleted T cells compared to the control, preferably by depleted CD8+ effector memory T-cells compared to the control.

7. The method according to any one of the foregoing claims, wherein said determining in step b) comprises determining whether said sample comprises a dark-zone phenotype;
wherein, optionally, said determining in step b) comprises determining whether said sample comprises
ii) a dark-zone phenotype;
iii) an enhanced signaling of TCF3, TCF4, and/or MYC compared to the control, preferably TCF3, TCF4, and MYC compared to the control;
iv) an enhanced expression of one or more, preferably a plurality of proteins selected from ribosomal proteins and ribosome-associated proteins, preferably ribosomal proteins, compared to the control; and
v) a tumor microenvironment **characterized by** depleted T cells compared to the control, preferably by depleted CD8+ effector memory T-cells compared to the control.

8. The method according to any one of the foregoing claims, wherein said enhanced signaling of TCF3, TCF4, MYC, BCR, MYD88, and/or PI3K compared to the control comprises an enhanced signaling of TCF4, MYC, BCR, and MYD88; TCF3, MYC, BCR, and PI3K; TCF3; TCF4; MYC; BCR; MYD88; PI3K; TCF3 and TCF4; TCF3 and MYC; TCF3 and BCR; TCF3 and MYD88; TCF3 and PI3K; TCF4 and MYC; TCF4 and BCR; TCF4 and MYD88; TCF4 and PI3K; MYC and BCR; MYC and MYD88; MYC and PI3K; BCR and MYD88; BCR and PI3K; MYD88 and PI3K; TCF3, TCF4, and MYC; TCF3, TCF4, and BCR; TCF3, TCF4, and MYD88; TCF3, TCF4, and PI3K; TCF3, MYC, and BCR; TCF3, MYC, and MYD88; TCF3, MYC, and PI3K; TCF3, BCR, and MYD88; TCF3, BCR, and PI3K; TCF3, MYD88, and PI3K; TCF4, MYC, and BCR; TCF4, MYC, and MYD88; TCF4, MYC, and PI3K; TCF4, BCR, and MYD88; TCF4, BCR, and PI3K; TCF4, MYD88, and PI3K; MYC, BCR, and MYD88; MYC, BCR, and PI3K; MYC, MYD88, and PI3K; BCR, MYD88, and PI3K; TCF3, TCF4, MYC, and BCR; TCF3, TCF4, MYC, and MYD88; TCF3, TCF4, MYC, and PI3K; TCF3, TCF4, BCR, and MYD88; TCF3, TCF4, BCR, and PI3K; TCF3, TCF4, MYD88, and PI3K; TCF3, MYC, BCR, and MYD88; TCF3, MYC, MYD88, and PI3K; TCF3, BCR, MYD88, and PI3K; TCF4, MYC, BCR, and PI3K; TCF4, MYC, MYD88, and PI3K; TCF4, BCR, MYD88, and PI3K; MYC, BCR, MYD88, and PI3K; TCF3, TCF4, MYC, BCR, and MYD88; TCF3, TCF4, MYC, BCR, and PI3K; TCF3, TCF4, MYC, MYD88, and PI3K; TCF3, TCF4, BCR, MYD88, and PI3K; TCF3, MYC, BCR, MYD88, and PI3K; TCF4, MYC, BCR, MYD88, and PI3K; or TCF3, TCF4, MYC, BCR, MYD88, and PI3K; preferably comprises an enhanced signaling of TCF4, MYC, BCR, and MYD88 or an enhanced signaling of TCF3, MYC, BCR, and PI3K;
wherein said enhanced signaling is indicative for the presence of a high-risk DLBCL in said patient.

9. The method according to any one of the foregoing claims, wherein said one or more, preferably a plurality of proteins are selected from PCNA, MCM6, NCL, PAICS, MYBBP1A, SSRP1, MCM4, CSDE1, MCM5, MCM3, PABPC4, SUPT16H, NASP, DDX21, BZW2, NAT10, PABPC1, RRP12, IGF2BP3, SMC4, LRPPRC, NOP2, POLD1, MTHFD1L, MKI67, FEN1, TOP2A, DDX18, PDCD11, PA2G4, SHMT2, CDC2, CCDC58, MSH2, NME1-NME2, HSPD1, GART, HSPA9, UBAP2, TOP1, MCM7, RRM1, RPL4, LYAR, GEMIN5, RRM2, GARS, IPO5, RUVBL1, GRPEL1, EEF2, NCAPH, RANBP1, IARS, RSL1D1, EEF1B2, SLIRP, NME1, ABCF2, PNPT1, MCM2, FKBP4, PBK, IMPDH2, ATXN10, DUT, TRAP1, RPL3, NOC2L, LDHA, MRTO4, YBX1, FARSB, SLC25A5, HNRNPA1, NUDC, HSP90AB1, KPNA2, ASNS, BAG2, NPM1, UTP18, TDP2, HSPE1, UTP14A, RPL35A, MARS, LDHB, RPS9, NOLC1, BTK, IMP4, NOL7, WDR3, PSMG1, SLC25A19, ALDH18A1, NIP7, CAD, C1QBP, NOL6, WDHD1, EEF1E1, NOB1, BTF3, MAD2L1, CYCS, PNO1, RPL26, TBRG4, WDR75, CACYBP, and combinations thereof.

10. The method according to any one of the foregoing claims, wherein said tumor microenvironment is **characterized by** depleted CD4+ T cells and/or depleted CD8+ T cells compared to the control; preferably by depleted follicular helper T cells and/or memory T cells compared to the control; more preferably by depleted CD8+ effector memory T-cells compared to the control.

11. The method according to any one of the foregoing claims, wherein said tumor microenvironment is further **characterized by** exhausted T cells compared to the control, preferably exhausted cytotoxic T cells, more preferably exhausted cytotoxic T cells of the EM2 and/or EM3 compartment.

12. The method according to any one of the foregoing claims, wherein said method comprises predicting a reduced time of progression-free survival and/or a reduced time of overall survival of said patient if said sample comprises at least two, preferably at least three, more preferably at least four, even more preferably all of features i)-v).

13. The method according to any one of the foregoing claims, wherein said method comprises stratifying said patient for an R-CHOP treatment, wherein said method comprises deciding in favor of or against said treatment based on the presence or absence of features i)-v),
wherein, if features i)-v) are absent in said sample, the decision is in favor of the R-CHOP treatment, and
wherein, if features i)-v) are present in said sample, the decision is against said R-CHOP treatment and optionally in favor of a treatment other than an R-CHOP treatment, such as a CAR T-cell therapy, a treatment with a bispecific antibody, and/or a treatment with venetoclax, ibrutinib, prednisone, obinutuzumab, and lenalidomide (ViPOR).

14. The method according to any one of the foregoing claims, wherein said determining in step b) comprises analyzing
i) said genotype using sequencing such as RNA sequencing, optionally single cell sequencing;
ii) said dark-zone phenotype using RNA sequencing;
iii) said enhanced signaling using RNA sequencing, mass spectrometry, single cell sequencing, or an immunoassay such as ELISA; preferably using RNA sequencing, mass spectrometry, or single cell sequencing;
iv) said one or more, preferably said plurality of proteins selected from ribosomal proteins and ribosome-associated proteins, preferably ribosomal proteins, using RNA sequencing, mass spectrometry, or an immunoassay such as ELISA; preferably using mass spectrometry; and
v) said tumor microenvironment using flow cytometry, mass cytometry, and/or sequencing such as single cell sequencing;
thereby obtaining biological data, wherein, optionally, said determining further comprises analyzing said biological data using a computer-implemented method which comprises a machine learning algorithm.

15. A kit for performing a method according to any one of the foregoing claims, the kit comprising means for determining at least two, preferably at least three, more preferably at least four, even more preferably all of features i)-v), and comprising instructions for performing the method according to any one of the foregoing claims; wherein, preferably, the kit comprises means for performing proteomics, transcriptomics, single cell sequencing, and/or an immunoassay; wherein, more preferably, the kit comprises means for performing RNA extraction, cDNA library preparation, and/or sequencing, and optionally further comprises instructions for data analysis.
